(19) **Europäisches Patentamt / European Patent Office / Office européen des brevets**

(11) **EP 4 421 456 A1**

(12) **EUROPEAN PATENT APPLICATION**
published in accordance with Art. 153(4) EPC

(43) Date of publication:
**28.08.2024 Bulletin 2024/35**

(21) Application number: **22882393.6**

(22) Date of filing: **02.08.2022**

(51) International Patent Classification (IPC):
*G01D 21/02* (2006.01)  *G01N 33/00* (2006.01)
*G01S 17/931* (2020.01)  *G01S 19/42* (2010.01)
*G06K 17/00* (2006.01)  *H04W 4/38* (2018.01)

(86) International application number:
**PCT/CN2022/109735**

(87) International publication number:
**WO 2023/065771 (27.04.2023 Gazette 2023/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.10.2021 CN 202111213708**

(71) Applicant: **Shanxi Quan'an New Technology Development Co., Ltd**
**Jiexiu City, Shanxi 032000 (CN)**

(72) Inventors:
• **GUO, Chunping**
  **Shanxi 032000 (CN)**
• **GUO, Xiaopeng**
  **Shanxi 032000 (CN)**

(74) Representative: **Meissner Bolte Partnerschaft mbB**
**Patentanwälte Rechtsanwälte**
**Postfach 86 06 24**
**81633 München (DE)**

(54) **REGULATION VIOLATION BEHAVIOR IDENTIFICATION METHOD AND INTELLIGENT ANTI-REGULATION VIOLATION SENSOR SYSTEM**

(57) The present invention relates to a regulation violation behavior identification method and an intelligent anti-regulation violation sensor system. In the present invention, dangerous source state information, operator regulation violation behavior action information, position and environment identification system information and other information are inputted into the intelligent anti-regulation violation sensor system, processed by a microprocessor and then transmitted to a 5G information converter, the 5G information converter then transmits the information to an anti-regulation violation cloud computing center, and the center is pre-installed with regulation violation behavior identification and processing software; the software comprises a regulation violation behavior identification algorithm, and the algorithm can determine regulation violation behavior types; according to the regulation violation behavior types, an execution mechanism is commanded to act, so that an alarm is given or a dangerous source is cut off. The intelligent anti-regulation violation sensor system comprises a lock mechanism regulation violation behavior identification system, a non-lock mechanism regulation violation behavior identification system, the 5G information converter, a computer or mobile phone terminal, the anti-regulation violation cloud computing center, an electric lock mechanism system, an identity identification system, an auxiliary variable E identification system, the microprocessor, a position and environment identification system, and a dangerous source information acquisition system.

```
┌─────────────────────────────────┐
│ obtain dangerous source state   │──S1210
│ information and behavior         │
│ information of an operator       │
└─────────────────────────────────┘
             │
             ▼
┌─────────────────────────────────┐
│ determine whether the operator  │──S1220
│ has a regulation violation       │
│ behavior according to the        │
│ dangerous source state           │
│ information and the behavior      │
│ information of the operator       │
└─────────────────────────────────┘
             │
             ▼
┌─────────────────────────────────┐
│ in a case where the operator     │──S1230
│ has the regulation violation      │
│ behavior, command an actuator     │
│ system to execute a first action  │
└─────────────────────────────────┘
```

Fig.12

EP 4 421 456 A1

## Description

### I. CROSS-REFERENCE TO RELATED APPLICATIONS

The present application is based on and claims the priority to the Chinese patent application No. 202111213708.1 filed on October 19, 2021, the disclosure of which is incorporated by reference herein in its entirety.

### II. TECHNICAL FIELD

The present disclosure pertains to the technical field of safety identification, and specifically relates to a method of identifying regulation violation behavior and an intelligent sensor system of preventing regulation violation.

### III. Background

Since the rise of Industrial Revolution, regulation violation behaviors and operations in industrial production are very common at home and abroad, which are mainly prevented all round the world by means of propaganda, training, education, management, laws and the like (i.e., so-called "huge-crowd strategy"), and although great achievements have been made, the regulation violation operations (including unsafe behaviors such as maloperations, which are all shortened to "regulation violations" herein) are still a main cause of various accidents. A study of a relevant institution in China noted that: 90%-95% of accidents are caused by regulation violation operations (unsafe behaviors). In order to thoroughly eradicate the regulation violation operations, a concept of preventing regulation violation technology was firstly proposed by the inventors in 2009 in an article published in a main scientific and technological journal, and a paper of "REGULATION VIOLATION BEHAVIOR ANALYSIS AND PREVENTING REGULATION VIOLATION COUNTERMEASURES" was completed by the inventors in 2017 in Peking University, so that theoretical support is provided for the preventing regulation violation technology. It is believed that, from no regulation violations to regulation violations, it would inevitably go through a safe "regulation violation transition process" that occupies certain space and time, and a dangerous operation can be performed after a "regulation violation red line" is crossed.

At present, in order to prevent underground regulation violation operations of a coal mine, the inventors of the present disclosure have applied for several Chinese and foreign patents, for example, the invention patent No. 200810055240.6 entitled "PRE-COVER-OPENING POWER-OFF METHOD AND APPARATUS" has been patented not only in China, but also in more than ten countries and organizations such as America, Russia, and European Union.

### IV. Summary

The present disclosure provides a regulation violation behavior identification method and an intelligent sensor system for preventing regulation violation.

According to one aspect of the present disclosure, there is provided a method of preventing regulation violation behavior, comprising: obtaining dangerous source state information and behavior information of an operator; determining whether the operator has a regulation violation behavior according to the dangerous source state information and the behavior information of the operator; and in a case where the operator has the regulation violation behavior, commanding an actuator system to execute a first action, for preventing the regulation violation behavior.

In some embodiments, the regulation violation behavior comprises at least one of: a regulation violation behavior of uncapping operation in an explosive environment, drowsy driving, a regulation violation behavior of off-duty, a regulation violation behavior of hazardous gas leakage, or a regulation violation behavior of entry to a dangerous area.

In some embodiments, in a case where the regulation violation behavior comprises the regulation violation behavior of uncapping operation in the explosive environment, the obtaining dangerous source state information and behavior information of an operator comprises at least one of: obtaining dangerous source state information of whether a target device is live and behavior information of whether the operator performs uncapping on the target device; or obtaining dangerous source state information of whether hazardous gas in an operation environment exceeds a limit threshold and behavior information of whether the operator performs uncapping on the target device.

In some embodiments, the determining whether the operator has a regulation violation behavior according to the dangerous source state information and the behavior information of the operator comprises: in a case where the dangerous source state information indicates that the target device is live or the hazardous gas in the operation environment exceeds the limit threshold, and the behavior information indicates that the operator performs uncapping on the target device, determining that the operator has the regulation violation behavior of the uncapping operation in the explosive environment; otherwise, determining that the operator does not have the regulation violation behavior of the uncapping operation in the explosive environment.

In some embodiments, the method further comprises: obtaining identity information of the operator; in a case where the operator has the regulation violation behavior, determining an identity of a violator according to the identity information of the operator; and in a case where the operator does not have the regulation violation behavior and identity authentication performed according to the identity information of the operator is passed, commanding the actuator system to execute a second action, for allowing the operation behavior.

According to one aspect of the present disclosure there is provided an apparatus of preventing regulation violation behavior, comprising: an obtaining module configured to obtain dangerous source state information and behavior information of an operator; a determining module configured to determine whether the operator has a regulation violation behavior according to the dangerous source state information and the behavior information of the operator; and a control module configured to, in a case where the operator has the regulation violation behavior, command an actuator system to execute a first action, for preventing the regulation violation behavior.

According to one aspect of the present disclosure, there is provided a system of preventing regulation violation behavior, comprising: an acquisition device configured to acquire dangerous source state information and behavior information of an operator; and a cloud computing center configured to: receive the dangerous source state information and the behavior information of the operator sent from the acquisition device; determine whether the operator has a regulation violation behavior according to the dangerous source state information and the behavior information of the operator; and in a case where the operator has the regulation violation behavior, command an actuator system to execute a first action, for preventing the regulation violation behavior.

In some embodiments, the regulation violation behavior comprises at least one of: a regulation violation behavior of uncapping operation in an explosive environment, drowsy driving, a regulation violation behavior of off-duty, a regulation violation behavior of hazardous gas leakage, or a regulation violation behavior of entry to a dangerous area.

In some embodiments, in a case where the regulation violation behavior comprises the regulation violation behavior of uncapping operation in the explosive environment, the acquisition device comprises: at least one of a first dangerous source state information acquisition device or a second dangerous source state information acquisition device, and an behavior information acquisition device, wherein: the first dangerous source state information acquisition device is configured to acquire dangerous source state information of whether a target device is live; the second dangerous source state information acquisition device is configured to acquire dangerous source state information of whether hazardous gas in an operation environment exceeds a limit threshold; and the behavior information acquisition device is configured to acquire behavior information of whether the operator performs uncapping on the target device.

In some embodiments, the behavior information acquisition device comprises: a sensor housing (303); a lock cylinder (305), which is arranged in a hole provided on one sidewall of the sensor housing (303) and fixes the sensor housing (303) onto a housing cover plate (313) of a device; a sensor circuit board (301), which is provided inside the sensor housing (303), in signal connection with a microswitch node or a Hall element active switch, and configured to, after detecting behavior information that the operator triggers the microswitch due to removing the lock cylinder and the sensor housing, send the behavior information to the cloud computing center.

In some embodiments, the behavior information acquisition device further comprises: a lossless connection sleeve (307), which is fixedly arranged on a bolt head of a fastening bolt (311) of the housing cover plate (313) of a device through a jackscrew (309), and configured to cooperate with the lock cylinder (305) to fix the sensor housing (303) onto the housing cover plate (313) of the device.

In some embodiments, the behavior information acquisition device comprises: a sensor housing (303), which is fixedly arranged on a device cover plate (525) and connected with a lock sleeve (527) to form an inner cavity of the sensor housing (303); an electric lock (517), which is arranged on the lock sleeve (527) and configured to, after identity identification performed by the cloud computing center for the operator is passed, act according to an instruction issued by the cloud computing center, to enable a key (519) to operate a lock cylinder (521) for unlocking to remove the sensor housing (303); and a sensor circuit board (301), which is arranged on the inner cavity of the sensor housing (303) and configured to, in a case where the sensor housing (303) is removed, send the behavior information to the cloud computing center.

In some embodiments, the behavior information acquisition device further comprises: an acousto-optic identification probe (509) and a card reader (510), which are connected with the sensor circuit board (301), the sensor circuit board (301) being further configured to send, to the cloud computing center, voice or image information of the operator obtained through the acousto-optic identification probe (509) or personal code information of a radio frequency card or a magnetic card obtained through the card reader (510), wherein the voice or image information of the operator or the personal code information is used for performing identity identification on the operator.

In some embodiments, the behavior information acquisition device further comprises: a lossless connection cover (523), which is fastened onto a device cover plate (525) through a lossless connection nut (512) and a lossless connection bolt (513); and a lossless connection bolt sleeve (515), which is sleeved onto the lossless connection bolt (513), the lock sleeve (527) being arranged on the lossless connection bolt sleeve (515) and fixed on the lossless connection bolt sleeve (515) through a lossless connection lock cylinder (521).

In some embodiments, the second dangerous source state information acquisition device comprises: a hazardous gas probe; and a leakage sensor for preventing regulation violation, which is configured to, in a case where the hazardous gas probe detects that concentration of the hazardous gas exceeds the limit threshold, send the dangerous source state information of the hazardous gas to the cloud computing center.

According to one aspect of the present disclosure, there is provided an apparatus of preventing regulation violation, comprising: a memory; and a processor coupled to the memory, the processor being configured to perform, based on instructions stored in the memory, the method of preventing regulation violation behavior as described above.

According to one aspect of the present disclosure, there is provided a computer-readable storage medium having thereon stored computer program instructions which, when executed by a processor, implement the method of preventing regulation violation behavior as described above.

According to one aspect of the present disclosure, there is provided a computer program, comprising: instructions which, when executed by a processor, cause the processor to perform the method of preventing regulation violation behavior as described above.

According to one aspect of the present disclosure, there is provided a method of identifying a regulation violation behavior, comprising: converting dangerous source state information into a value of a variable X by a dangerous source identification system, or converting behavior of an operator into a value of a variable B1 by a regulation violation behavior identification system of device-based lock mechanism, or converting behavior of the operator into a value of a variable B2 by regulation violation behavior identification system based on a non-lock mechanism arranged in an operating place, or converting identity information of the operator into a value of a variable C by an identity identification system, or converting information of position of the operator and information of surrounding environment of the operator into a value of a variable D by a position and environment identification system, or converting a state comprising voltage and current auxiliary variables into a value of a variable E by an auxiliary variable E identification system, or processing action state of an electric lock mechanism system by a microprocessor and storing a value of a logical variable H in a cloud computing center for preventing regulation violation, wherein at least one of values of the variables X, B1, B2, C, D, E, or H is formed by being processed by a microprocessor of an intelligent sensor system for preventing regulation violation and then transmitted to a 5G information converter and then transmitted to the cloud computing center for preventing regulation violation pre-installed with identification and processing software for preventing regulation violation behavior, or the values of the variables are directly called and shared by the cloud computing center for preventing regulation violation from other data system; determining types of regulation violation behavior by the cloud computing center for preventing regulation violation, through computing the variables X,B1,B2,C,D,E and H according to an identification algorithm of preventing regulation violation behavior; transmitting the types back to the 5G information converter; processing the types by the microprocessor, for commanding an actuator system to act, giving an alarm, or cutting off a dangerous source or locking the dangerous source.

In some embodiments, the dangerous source identification system comprises a dangerous source identification and locking system for a power supply system, an identification system for identifying a drowsiness dangerous source upon a regulation violation behavior of drowsiness, an identification system for identifying a not-watching-computer dangerous source upon a regulation violation behavior of not-watching-computer, an identification system for identifying hazardous gas methane dangerous source, an identification system for identifying a regulation violation behavior of entering a dangerous area, an identification system for identifying unmanned-vehicle (905) dangerous source, and an identification system for identifying regulation violation behavior of entering a rail transport lane where a pedestrian is not admitted.

The dangerous source identification and locking system for the power supply system comprises: a feed sensor based on the power supply system, or a photoelectric voltage converter of a breaker, or a voltage sensor, or a voltage analog-to-digital converter circuit for a monitoring system, or a voltage dangerous source identification system for a switch comprehensive protection means. The dangerous source identification and locking system converts dangerous voltage of supply power into safe voltage through a voltage conversion circuit. The safe voltage is transmitted to the microprocessor through a port of a dangerous source information acquisition system of the intelligent sensor system for preventing regulation violation, processed by the microprocessor, and then sent from the 5G information converter to the cloud computing center for preventing regulation violation, to form a voltage dangerous source logical variable X1. In a case where there is dangerous voltage, X1=1, otherwise X1=0.

The identification system for identifying a drowsiness dangerous source upon a regulation violation behavior of drowsiness comprises a conveyor magnetic starter (601), a drowsiness sensor (603) for preventing regulation violation based on non-lock identification, and the cloud computing center (7) for preventing regulation violation, wherein: the drowsiness sensor (603) is arranged on the conveyor magnetic starter (601) controlling a conveyor; a switch state signal of the conveyor magnetic starter (601) is transmitted to the drowsiness sensor (603) through a connecting wire (602); drowsiness sensor (603) transmits the switch state signal for controlling startup or shutdown of the conveyor by the conveyor magnetic starter (601), which is acting as dangerous source state information of the system, to the microprocessor (15) through a port of a dangerous source information acquisition system of the intelligent sensor system for preventing regulation violation; the switch state signal is processed by the microprocessor (15) and then transmitted from a 5G information converter (5) to the cloud computing center (7) for preventing regulation violation, to form a drowsiness dangerous source logical variable X2.In a case where the switch state signal is startup, X2=1.In a case where the switch state signal is shutdown, X2=0.

The identification system for identifying a not-watching-computer dangerous source upon a regulation violation behavior of not-watching-computer comprises a device startup/shutdown state data system monitored by a computer and the cloud computing center (7) for preventing regulation violation, wherein: the system is networked with the cloud computing center (7) for calling and sharing the startup/shutdown dangerous source state information; the startup/shutdown dangerous source state information is sent to the cloud computing center (7), and then converted into a logical variable X3.In a case where the dangerous source state information is startup, X3=1, otherwise X3=0.

The identification system for identifying hazardous gas dangerous source comprises: a hazardous gas probe, and a manual valve leakage sensor (701) for preventing regulation violation based on non-lock identification connected therewith, wherein in a case where a concentration value measured by the hazardous gas probe exceeds a specified value, the concentration value is transmitted to the microprocessor (15) through a port of a dangerous source information acquisition system of the intelligent sensor system for preventing regulation violation, processed by the microprocessor (15), and then sent from the 5G information converter (5) to the cloud computing center (7) for preventing regulation violation, to form a hazardous gas dangerous source logical variable X4. In a case where the concentration value exceeds the specified value, X4=1, otherwise X4=0.

The identification system for identifying hazardous gas methane dangerous source comprises: a mine methane monitoring system, wherein: the mine methane monitoring system is networked with the cloud computing center (7) for preventing regulation violation through a ground scheduling station; a measured methane dangerous source concentration value is directly sent to the ground scheduling station; the cloud computing center (7) for preventing regulation violation is networked with the ground scheduling station for calling and sharing the methane dangerous source concentration value; and the cloud computing center (7) for preventing regulation violation is configured to form a methane dangerous source logical variable X5 through comparison computation. In a case where the methane dangerous source concentration value exceeds a limit, X5=1, otherwise X5=0.

The identification system for identifying a regulation violation behavior of entering a dangerous area comprises: a regulation violation red line (803) and an entry sensor (811) for preventing regulation violation based on non-lock identification, wherein: the entry sensor (811) with a human body sensing probe is arranged on the regulation violation red line (803); and the entry sensor (811) continuously sends dangerous source information of the dangerous area, which is acting as regulation violation red line marker information, to the cloud computing center (7) for preventing regulation violation, to form a logical variable X6. In a case where the regulation violation red line marker information is received, X6=1, otherwise X6=0.

The identification system for identifying unmanned vehicle dangerous source comprises: a dangerous source speed probe and a crash sensor (900) for preventing regulation violation based on non-lock identification, wherein: the dangerous source speed probe and the crash sensor (900) are arranged on an unmanned vehicle (905), and the crash sensor (900) transmits dangerous source information of the unmanned vehicle (905) to the microprocessor (15) through a port of a dangerous source information acquisition system of the intelligent sensor system for preventing regulation violation; the dangerous source information is processed by the microprocessor (15), and then sent from the 5G information converter (5) to the cloud computing center (7) for preventing regulation violation, to form a moving vehicle dangerous source logical variable X7. In a case where a speed reaches a danger value, the logical variable X7=1, otherwise, X7=0.

The identification system for identifying a regulation violation behavior of entering a rail transport lane where a pedestrian is not admitted comprises: a vehicle power control switch (107) of the rail transport and a pedestrian sensor (105) for preventing regulation violation based on non-lock identification, wherein: the pedestrian sensor (105) is arranged and connected on the vehicle power control switch (107); the pedestrian sensor (105) is configured to transmit rail transport dangerous source information to the microprocessor (15) through a port of a dangerous source information acquisition system of the intelligent sensor system for preventing regulation violation; the rail transport dangerous source information is processed by the microprocessor (15) and sent from the 5G information converter (5)to the cloud computing center (7) for preventing regulation violation; and the cloud computing center (7) for preventing regulation violation takes an on/off state signal of the vehicle power control switch (107) as a dangerous source logical variable X8. In a case where the switch is on, it is indicated that the rail transport vehicle (103) is in danger of crash into a person during moving, X8=1, otherwise, X8=0.

Further, the regulation violation behavior identification system of device-based lock mechanism comprises: a sensor circuit board (301), a sensor housing (303), a lock cylinder (305), a lossless connection sleeve (307), and a jackscrew (309), wherein: the lossless connection sleeve (307) is arranged on a bolt head of a fastening bolt (311) of a housing cover plate (313) of a device and then fixed onto a head of the fastening bolt (311) with the jackscrew (309); the sensor circuit board (301) is provided on one side inside the sensor housing (303); the sensor circuit board (301) is in signal connection with a microswitch node or a Hall element active switch; the other side inside the sensor housing (303) is arranged on the lossless connection sleeve (307); the lock cylinder (305) is arranged in a hole provided on one sidewall of the sensor housing (303), and fixes the sensor housing (303) onto the housing cover plate (313)of the device.

In a case where a full-time person removes the lock cylinder (305) by using a special key and then removes the sensor housing (303), the microswitch on the sensor circuit board (301) is triggered, action information of the switch is processed by the sensor circuit board (301), sent to a mining cloud computing center (201) for preventing regulation violation, processed and converted into a behavior logical variable B11, wherein: in a case where the sensor housing is removed, $B11=1$, otherwise $B11=0$.

Or the regulation violation behavior identification system of device-based lock mechanism comprises: a sensor circuit board (301), a sensor housing (303), a lock cylinder (521), a lossless connection nut (512), a lossless connection bolt (513), a lossless connection bolt sleeve (515), a lossless connection cover (523), and a lock sleeve (527), wherein: the lossless connection cover (523) is arranged on a device cover plate (525); the lossless connection nut (512) is fixed onto the lossless connection cover (523); the lossless connection bolt (513) is sleeved with the lossless connection bolt sleeve (515) and screwed into the lossless connection nut (512) to fasten the lossless connection cover (523) onto the device cover plate (525); the lock sleeve (527) is arranged onto the lossless connection bolt sleeve (515); the lock sleeve (527) is fixed onto the lossless connection bolt sleeve (515) by screwing a key (519) into the lock cylinder (521); the sensor housing (303) is connected with the lock sleeve (527) to form an inner cavity of the sensor housing (303); the sensor circuit board (301) is arranged on the inner cavity of the sensor housing (303); an electric lock (517) of an electromagnet or a stepping motor is arranged on the lock sleeve (527); an acousto-optic identification probe (509) and a card reader (510) are arranged on one side surface of the sensor housing (303); and the sensor circuit board (301) is connected with the electric lock (517), the acousto-optic identification probe (509), and the card reader (510).

After the sensor circuit board (301) obtains voice or an image of an operator through the acousto-optic identification probe (509), or obtains personal code of a radio frequency card or a magnetic card through the card reader (510), the voice or image or personal code is transmitted to the microprocessor through a port of the regulation violation behavior identification system of device-based lock mechanism of the intelligent sensor system for preventing regulation violation, and sent from a 5G information converter to the mining cloud computing center (201) for preventing regulation violation. The mining cloud computing center (201) for preventing regulation violation enables the regulation violation behavior identification software for computation. In a case where the operator is determined to be a full-time person, an instruction issued by the mining cloud computing center (201) is transmitted from the 5G information converter to the microprocessor (15) for processing, for commanding the electric lock (517) to act to open a passage, to enable the key (519) to operate the lock cylinder (521) for unlocking to remove the sensor housing (303). In a case where the sensor housing (303) is removed, information is transmitted from the sensor circuit board (301) to the mining cloud computing center (201) for preventing regulation violation, and converted into an behavior logical variable B12. In a case where the sensor housing is removed, $B12=1$, otherwise $B12=0$.

In some embodiments, the regulation violation behavior identification system based on non-lock mechanism comprises an identification system for identifying a regulation violation behavior of drowsiness, an identification system for identifying a regulation violation behavior of not-watching-computer, an identification system for identifying regulation a regulation violation behavior of hazardous gas leakage, an identification system for identifying a regulation violation behavior of entering a dangerous area, an identification system for identifying a regulation violation behavior of an unmanned vehicle (905), and an identification system for identifying a regulation violation behavior of entering a rail transport lane where a pedestrian is not admitted.

The identification system for identifying a regulation violation behavior of drowsiness comprises: a drowsiness sensor (603)for preventing regulation violation based on non-lock identification and an preventing drowsiness means (607), wherein: the drowsiness sensor (603) is arranged on a conveyor magnetic starter (601) controlling a conveyor; the conveyor magnetic starter (601) is connected with the drowsiness sensor (603) through a connecting wire (602); the drowsiness sensor (603) is connected with the preventing drowsiness means (607) and wirelessly connected with the cloud computing center (7) for preventing regulation violation; and the cloud computing center (7) for preventing regulation violation is directly connected with a personnel positioning system (611) for data calling and sharing. In a case where the operator works normally, the preventing drowsiness means (607) is operated regularly. In a case where the operator is incapable of working normally due to regulation violation behavior of drowsiness, the operation of the preventing drowsiness means (607) is automatically stopped and drowsiness state information is sent to the drowsiness sensor (603), processed by the microprocessor (15) and sent from the 5G information converter (5) to the cloud computing center (7), to form the drowsiness logical variable B21. In a case where the operator is drowsy, $B21=1$, otherwise $B21=0$.

The identification system for identifying a regulation violation behavior of not-watching-computer comprises a not-watching-computer probe for preventing a regulation violation behavior, the not-watching-computer probe being capable of regularly sending a sensing signal, wherein the not-watching-computer probe capable of regularly sending a sensing signal is arranged on a computer and connected with the intelligent sensor system for preventing regulation violation. In a case where an operator observes regulation to watch computer, the not-watching-computer probe regularly sends a signal to the intelligent sensor system for preventing regulation violation. In a case where the operator violates regulation not to watch computer, the not-watching-computer probe sends another signal, which is processed by the microprocessor (15) and transmitted from the 5G information converter (5) to the cloud computing center for preventing regulation violation, to form a logical variable B22 after comparison computation. In a case where the operator does not watch computer, B22=1, otherwise B22=0.

The identification system for identifying a regulation violation behavior of hazardous gas leakage comprises: a manual valve leakage sensor (701) for preventing regulation violation based on non-lock identification and an electric valve leakage sensor (709) for preventing regulation violation based on non-lock identification, wherein: the manual valve leakage sensor (701) and the electric valve leakage sensor (709) are arranged at two positions and are both connected to the cloud computing center (7) for preventing regulation violation through their 5G information converters (5) for wireless communication; and the manual valve leakage sensor (701) is connected to one hazardous gas probe and one human body sensing probe. In a case where time of hazardous gas leakage caused by regulation violation is more than a certain time, the human body sensing probe of the manual valve leakage sensor (701) transmits information of whether there is an operator on site to the microprocessor (15). The information is processed and wirelessly reported to the cloud computing center (7) for preventing regulation violation for comparison computation, to form a logical variable B23. In a case where an operator is on site, B23=1, otherwise B23=0.

The identification system for identifying a regulation violation behavior of entering a dangerous area comprises: a regulation violation red line (803) and an entry sensor (811) for preventing regulation violation based on non-lock identification, wherein the entry sensor (811) is arranged on the regulation violation red line. In a case where the operator touches the regulation violation red line (803), the entry sensor (811) sends regulation violation behavior information of entering a dangerous area to the cloud computing center (7) for preventing regulation violation through a 5G information converter (5) corresponding to the entry sensor, to form a logical variable B24. In a case where the operator touches the regulation violation red line, B24=1, otherwise B24=0.

The identification system for identifying a regulation violation behavior of an unmanned vehicle comprises: a human body sensing radar probe and a crash sensor (900) for preventing regulation violation based on non-lock identification, wherein: the human body sensing radar probe is arranged on the crash sensor (900); and the human body sensing radar probe and the crash sensor (900) are arranged on an unmanned vehicle (905); the crash sensor (900) transmits, to the microprocessor, behavior information of a pedestrian (903) who violates regulation to enter a road that is detected by the human body sensing radar probe; the behavior information is sent from the 5G information converter to the cloud computing center (7) for preventing regulation violation, to form a regulation violation logical variable B25. In a case where a distance between the pedestrian (903) who violates regulation to enter a road and the unmanned vehicle (905) approaches a specified value, B25=1, otherwise B25=0.

The identification system for identifying a regulation violation behavior of entering a rail transport lane where a pedestrian is not admitted comprises: a vehicle power control switch (107), a pedestrian sensor (105) for preventing regulation violation based on non-lock identification, and a laser (109), wherein: the pedestrian sensor (105) is arranged on the vehicle power control switch (107) and connected with the laser (109); laser emitted by the laser (109) is a regulation violation red line. In a case where a person (100) who violates regulation to enter a rail transport lane performs regulation violation behavior of shielding the laser, pedestrian sensor (105) transmits regulation violation behavior information of the person to the microprocessor, the regulation violation behavior information being sent from the 5G information converter to the cloud computing center (7) for preventing regulation violation, to form a rail pedestrian regulation violation logical variable B26. In a case where the regulation violation red line is touched, B26=1, otherwise B26=0.

In some embodiments, the identity identification system (11) comprises: an acoustic-optical identification probe (509), a card reader (510), and information of voice, an image, or a radio frequency card number profile identity of a full-time person that is pre-installed in the cloud computing center for preventing regulation violation, wherein: the acoustic-optical identification probe (509) and the card reader (510) are connected with the sensor circuit board; the acoustic-optical identification probe (509) and the card reader (510) transmit acquired actual identity information of the operator to the microprocessor (15); the acquired actual identity information is processed by the microprocessor, and then sent from the 5G information converter (5) to the cloud computing center (7) for preventing regulation violation, and then compared with the profile information of the full-time person that is pre-installed in the cloud computing center (7) for preventing regulation violation to identify an operator identity, to form a logical variable C. In a case where the operator is full-time person, C=1, otherwise C=0. An identification result C=1 or C=0 is transmitted from the 5G information converter (5) to the microprocessor (15) to command the electric lock of the electric lock mechanism system (9) to act.

In some embodiments, the position and environment identification system comprises a database of the cloud computing center (7) for preventing regulation violation where information of device performance and sensor position profile information are stored, and an environmental temperature and photosensitive probe device arranged and connected on the intelligent sensor system for preventing regulation violation, wherein: the position and environment identification system regularly acquires related parameter information of a sensor position and a device surrounding temperature and transmits the information to the microprocessor (15); the information is sent from the 5G information converter to the cloud computing center (7) for preventing regulation violation, to form a position and environment identification logical variable D; and after the cloud computing center (7)for preventing regulation violation performs analysis and computation, a result of the analysis and computation is sent to a computer or a mobile phone terminal (6). In a case where the related parameter information is not zero, D=1, otherwise D=0.

The auxiliary variable E identification system (13) comprises a switch comprehensive protection means and a radio frequency card reader, wherein: the auxiliary variable E identification system regularly acquires related parameter information of device voltage, device current, device short-circuit protection setting value, device leakage protection action, device power factor, and personnel patrol inspection, and transmits the information to the microprocessor (15); the information is processed and then transmitted from the 5G information converter to the cloud computing center (7) for preventing regulation violation, to form an auxiliary variable logical variable E; and after analysis and computation by the cloud computing center (7) for preventing regulation violation, a result of the analysis and computation is transmitted to a computer or a mobile phone terminal (6). In a case where the related parameter information of the auxiliary variable logical variable E is not zero, E=1, otherwise E=0.

In some embodiments, the actuator system is any of the electric lock mechanism system (9), a superior power switch sensor (215)for preventing regulation violation, a pipeline electric valve (707), a possible rear-ending vehicle (907), a vehicle power control switch (107), or a mobile phone of an emergency rescuer (807).

The electric lock mechanism system (9) comprises an electro-magnet electric lock or stepping motor electric lock, wherein the electro-magnet electric lock or stepping motor electric lock (517) is arranged on a lock sleeve (527). An electric lock action state signal is sent to the cloud computing center (7) for preventing regulation violation through the microprocessor (15) and the 5G information converter (5) in succession, to form a logical variable H. In a case where the electric lock is opened, H=1. In a case where the electric lock is closed, H=0. The cloud computing center (7) for preventing regulation violation performs identification computation after receiving a signal sent from the identity identification system (11). In a case where the person is considered to be a full-time person, H=1 is set and the electric lock is commanded to be opened; otherwise, H=0 is set and the electric lock is commanded to be closed.

The superior power switch sensor (215) for preventing regulation violation is arranged on a load switch (209), wherein a load switch sensor (205) for preventing regulation violation issues a power cut or locking request to an superior power switch (213) controlling a dangerous source facility. The request is computed and processed by a mining cloud computing center (201) for preventing regulation violation, for commanding the superior power switch sensor (215) to issue a power cut instruction, to command the superior power switch (213) to be powered off or locked.

While the superior power switch (213) transmits power to the load switch (209), live information is transmitted from the superior power switch sensor (215) to the mining cloud computing center (201)for preventing regulation violation, and converted into a voltage dangerous source logical variable X1. In a case where there is live, X1=1, otherwise X1=0. In a case where X1=1, the mining cloud computing center (201) for preventing regulation violation synchronously commands an electric lock of the load switch sensor (205) for preventing regulation violation to be locked, to disable the operator to perform a regulation violation behavior of uncapping operation, for identifying and locking a live dangerous source. In a case where X1=0, the mining cloud computing center (201) for preventing regulation violation synchronously commands the electric lock of the load switch sensor (205) to be unlocked, to enable the operator to perform an uncovering operation.

The pipeline electric valve (707), the possible rear-ending vehicle (907), the vehicle power control switch (107), or the mobile phone of the emergency rescuer (807) is a facility for controlling the dangerous source. After the cloud computing center (7) for preventing regulation violation determines a regulation violation and sends an instruction to the facility for controlling the dangerous source, the facility for controlling the dangerous source is commanded to act, to cut off and lock the dangerous source, and meanwhile, give an alarm.

In some embodiments, the regulation violation identification algorithm comprises at least one of:

$$W11=X1*(B1+B2)*C=1;$$

$$W12=X2*(B1+B2)*C=1;$$

$$W1n=Xn*(B1+B2)*C=1;$$

$$W21=X1*(B1+B2)=1;$$

$$W22=X2*(B1+B2)=1;$$

$$W2n=Xn*(B1+B2)=1;$$

$$W3=f(X,B1,B2,C,D,E,H)=1;$$

or

$$FW=(B1+B2)*FX*FC=1;$$

where X1, X2 and Xn represent first, second and nth dangerous source logical variables, W with a serial number represents various regulation violation behavior logical variables, FW represents a non-regulation violation behavior logical variable, FX represents a non-dangerous source logical variable, and FC represents a non-full-time person logical variable.

In some embodiments, the cloud computing center for preventing regulation violation is further capable of being networked with BeiDou Navigation System, GPS Navigation System, an underground personnel positioning system, or a methane monitoring system, for data calling and sharing.

According to another aspect of the present disclosure, the present disclosure provides an intelligent sensor system for preventing regulation violation, which performs a method of identifying a regulation violation behavior, comprising a regulation violation behavior identification system (1) based on lock mechanism, a regulation violation behavior identification system (3) based on non-lock mechanism, a 5G information converter (5), a computer or a mobile phone terminal (6) installed with regulation violation behavior identification and processing software, a cloud computing center (7) for preventing regulation violation installed with the regulation violation behavior identification and processing software, an electric lock mechanism system (9), an identity identification system (11), an auxiliary variable E identification system (13), a microprocessor (15), a position and environment identification system (17), and a dangerous source information acquisition system (19), wherein: an information input end of the microprocessor (15) is connected with the regulation violation behavior identification system (1) based on lock mechanism, the regulation violation behavior identification system (3) based on non-lock mechanism, the dangerous source information acquisition system (19), the identity identification system (11), the position and environment identification system (17), and the auxiliary variable E identification system (13); a control signal output end of the microprocessor (15) is connected with signal input ends of the electric lock mechanism system (9) and the 5G information converter (5); a signal output end of the 5G information converter (5) is wirelessly connected with the cloud computing center (7) for preventing regulation violation; and the cloud computing center (7) for preventing regulation violation is connected with the computer or the mobile phone terminal (6).

A basic workflow of the intelligent sensor system for preventing regulation violation comprises: starting; initializing; reading EEPROM configuration information; configuring the 5G information converter (5); connecting a 5G network; receiving an command from the cloud computing center (7) for preventing regulation violation, and parsing the command; regularly reporting information of the dangerous source information acquisition system (19), information of the position and environment identification system (17), and information of the auxiliary variable E identification system (13); and circularly detecting information of the regulation violation behavior identification system (1) based on lock mechanism, information of the regulation violation behavior identification system (3) based on non-lock mechanism, information of the identity identification system (11), and electric lock action information of the electric lock mechanism system (9).

## V. Brief description of the drawings

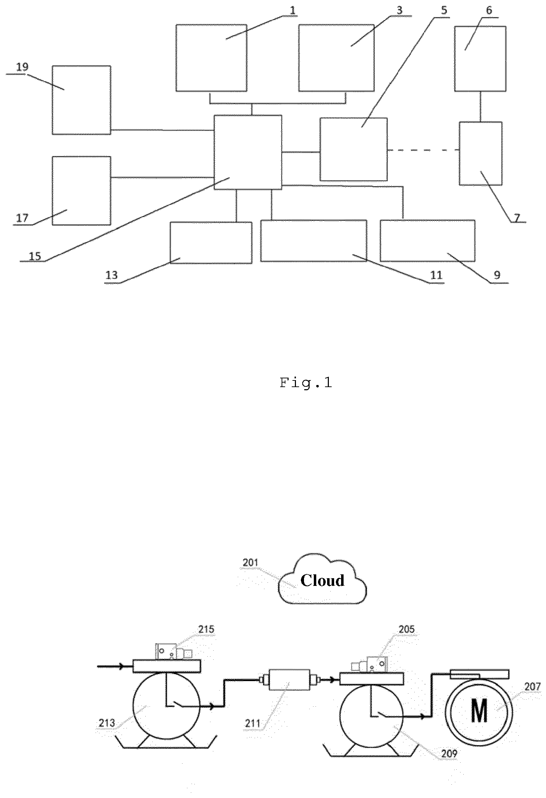

Fig. 1 is a schematic structural diagram of an intelligent sensor system for preventing regulation violation according to some embodiments of the present disclosure,
where 1- regulation violation behavior identification system based on lock mechanism, 3- regulation violation behavior identification system based on non-lock mechanism, 5-5G information converter, 6- computer or mobile phone terminal, 7- cloud computing center for preventing regulation violation, 9-electric lock mechanism system, 11-identity identification system, 13-auxiliary variable E identification system, 15-microprocessor, 17-position and environment identification system, and 19-dangerous source information acquisition system.

Fig. 2 is a schematic diagram of an identification system for a regulation violation behavior of live operation in an explosive environment according to some embodiments of the present disclosure,
where 201-mining cloud computing center for preventing regulation violation, 205-load switch sensor for preventing regulation violation, 207-load, 209-load switch, 211-cable junction box, 213-superior power switch, 215-superior power switch sensor for preventing regulation violation.

Fig. 3 is a schematic diagram of a regulation violation identification system based on mechanical lock lossless connection mechanism according to some embodiments of the present disclosure.

Fig. 4 is a schematic cross-sectional view of a lossless connection sleeve, a jackscrew, and a fastening bolt in a regulation violation identification system based on mechanical lock lossless connection mechanism according to some embodiments of the present disclosure,
where 301-circuit board of an intelligent sensor system for preventing regulation violation, a sensor circuit board for short, 303-sensor housing, 305-lock cylinder, 307-lossless connection sleeve, 309-jackscrew, 311-fastening bolt, 313- housing cover plate of a device, and 315-locking notch.

Fig. 5 is a schematic diagram of a regulation violation identification system based on an electric lock according to some embodiments of the present disclosure,
where 509-acousto-optic identification probe, 510-card reader, 512-lossless connection nut, 513-lossless connection bolt, 515-lossless connection bolt sleeve, 517-electric lock, 519-lock key; 521-lossless connection lock cylinder; 523-lossless connection cover; 525-device cover plate; 527-lock sleeve.

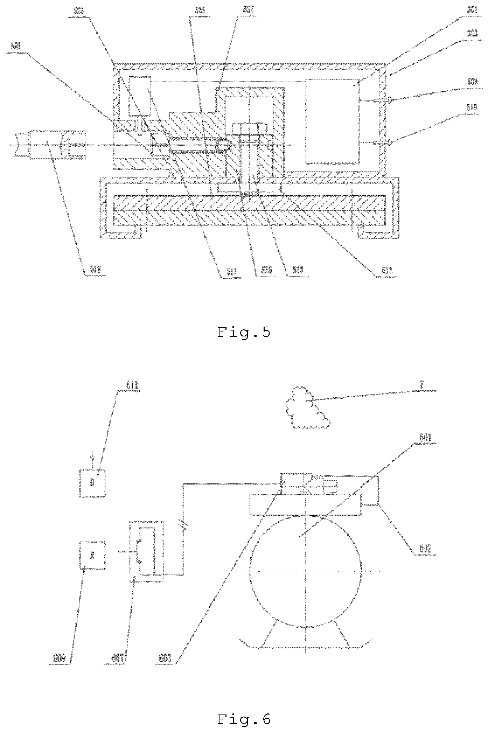

Fig. 6 is a schematic diagram of a system for preventing a conveyor driver from drowsing in violation of regulations according to some embodiments of the present disclosure,
where 601-conveyor magnetic starter, 602-connecting wire, 603- drowsiness sensor for preventing regulation violation based on non-lock identification, 607-preventing drowsiness means, 609-belt conveyor driver, 611-personnel positioning system.

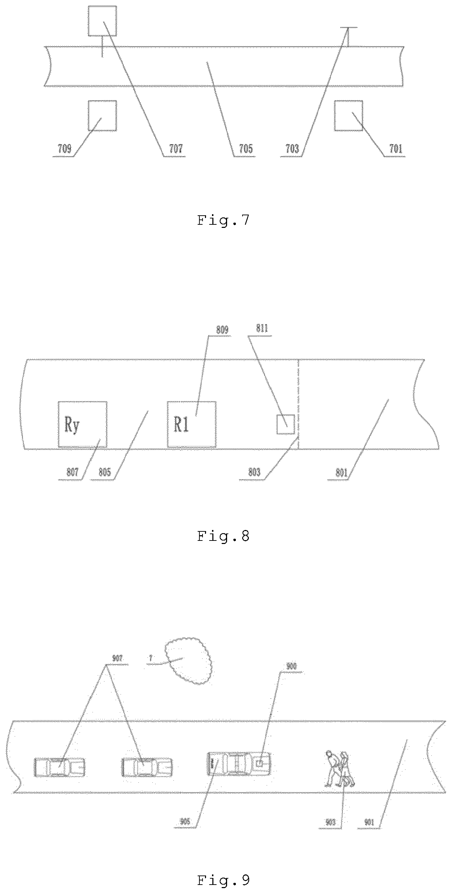

Fig. 7 is a schematic diagram of an identification system for identifying a regulation violation behavior of hazardous gas leakage according to some embodiments of the present disclosure,
where 701-manual valve leakage sensor for preventing regulation violation based on non-lock identification, 703-manual valve, 705-hazardous gas pipeline, 707-pipeline electric valve, and 709-electric valve leakage sensor for preventing regulation violation based on non-lock identification.

Fig. 8 is an identification system for identifying a regulation violation behavior of entering a dangerous area according to some embodiments of the present disclosure,
where 801-dangerous area, 803-regulation violation red line, 805-underground safe operation area, 807-emergency rescuer, 809-dangerous area operator, 811- entry sensor for preventing regulation violation based on non-lock identification.

Fig. 9 is a schematic diagram of an identification system for identifying a violator based an unmanned vehicle according to some embodiments of the present disclosure,
where 900- crash sensor for preventing regulation violation based on non-lock identification, 901-road, 903-pedestrian of regulation violation entry to a road; 905-unmanned vehicle, 907-possible rear-ending vehicle.

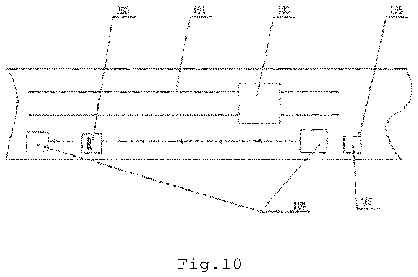

Fig. 10 is an identification system for identifying a regulation violation behavior of entering a rail transport lane where a pedestrian is not admitted according to some embodiments of the present disclosure,
where 100-person of regulation violation entry to a rail transport lane; 101-rail of rail transport lane, 103-rail transport vehicle, 105- pedestrian sensor for preventing regulation violation based on non-lock identification, 107-vehicle power control switch, 109-laser.

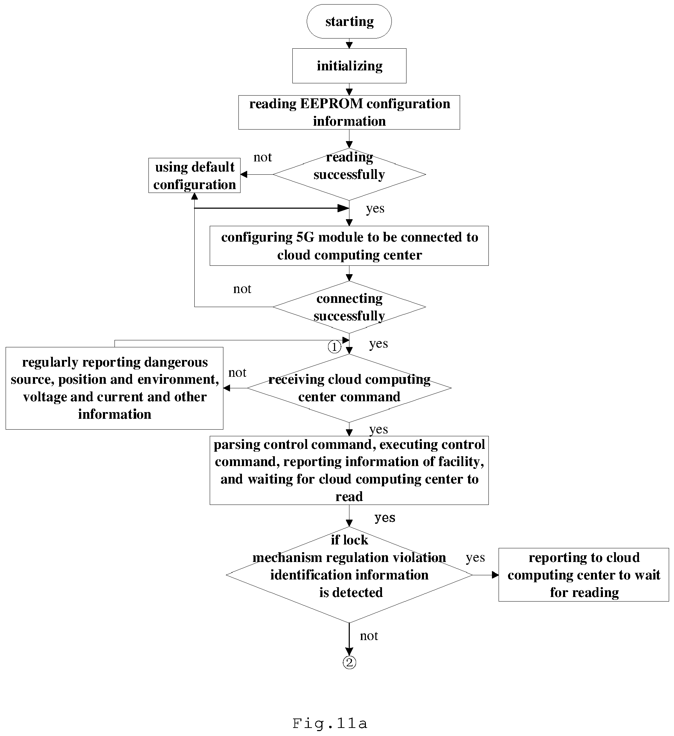

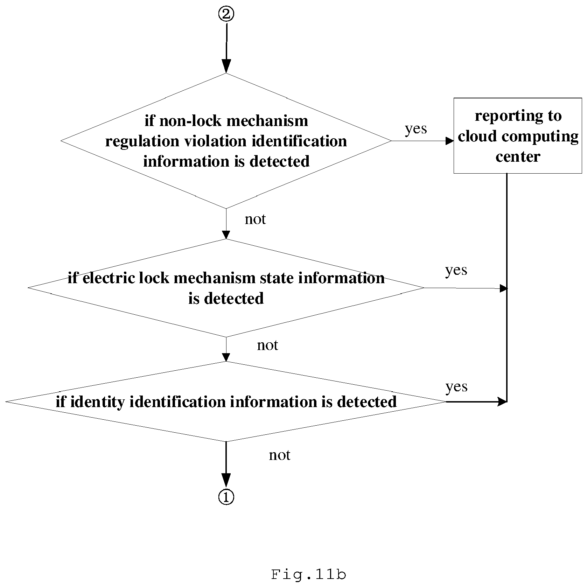

Figs.11a and 11b are basic workflows of an intelligent sensor system for preventing regulation violation according to some embodiments of the present disclosure.

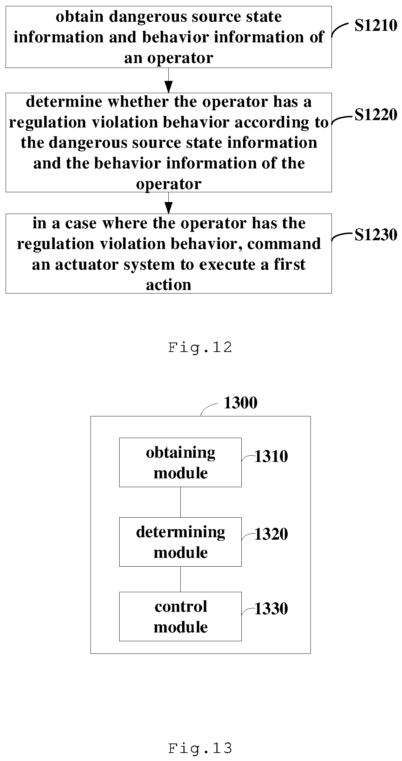

Fig. 12 is a schematic flow diagram of a method of preventing regulation violation behavior according to some embodiments of the present disclosure.

Fig. 13 is a schematic structural diagram of an apparatus of preventing regulation violation behavior according to some embodiments of the present disclosure.

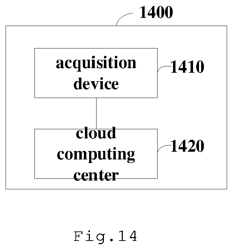

Fig. 14 is a schematic structural diagram of a system of preventing regulation violation behavior according to some embodiments of the present disclosure.

## VI. Detailed description

Although an apparatus for preventing regulation violation in the related art can prevent a regulation violation behavior, it has a defect of being unable to accurately identify the regulation violation behavior.
In view of this, an objective of the present disclosure is to solve the technical problem of being unable to accurately identify the regulation violation behavior that exists in the existing apparatus for preventing regulation violation.
The present disclosure will be further described in conjunction with the accompanying drawings and embodiments.
As shown in Fig. 1, an intelligent sensor system for preventing regulation violation for a method of identifying a regulation violation behavior in some embodiments of the present disclosure comprises a regulation violation behavior identification system 1 based on lock mechanism, a regulation violation behavior identification system 3 based on non-lock mechanism, a 5G information converter 5, a computer or a mobile phone terminal 6 installed with regulation violation behavior identification and processing software, a cloud computing center 7 for preventing regulation violation installed with the violation behavior identification and processing software, an electric lock mechanism system 9, an identity identification system 11, an auxiliary variable E identification system 13, a microprocessor 15, a position and environment identification system 17, and a dangerous source information acquisition system 19, wherein an information input end of the microprocessor 15 is connected with the regulation violation behavior identification system 1 based on lock mechanism, the regulation violation behavior identification system 3 based on non-lock mechanism, the dangerous source information acquisition system 19, the identity identification system 11, the position and environment identification system 17, and the auxiliary variable E identification system 13; a control signal output end of the microprocessor 15 is connected with signal input ends of the electric lock mechanism system 9 and the 5G information converter 5; a signal output end of the 5G information converter 5 is wirelessly connected with the cloud computing center 7 for preventing regulation violation; and the cloud computing center 7 for preventing regulation violation is connected with the computer or mobile phone terminal 6.

Where the regulation violation behavior identification system 1 based on lock mechanism comprises a regulation violation behavior identification system based on electric lock mechanism and a regulation violation behavior identification system based on mechanical lock lossless connection mechanism. Information output by the system is processed by the microprocessor 15.A behavior logical variable transmitted and stored in the cloud computing center 7 for preventing regulation violation is represented by B1.

The regulation violation behavior identification system 3 based on non-lock mechanism comprises: a "regulation violation red line" which is set according to a specific safety standard requirement and not allowed to be touched, and video camera (camera), a laser sensor, a drowsiness means for preventing regulation violation or the like manually set on the regulation violation red line (beside the regulation violation red line and within a range defined by the regulation violation red line). In a case where an operator touches the regulation violation red line, action information is output by a sensing device, processed by the microprocessor 15. A logical variable transmitted and stored in the cloud computing center 7 for preventing regulation violation is represented by B2.

The 5G information converter 5 comprises various 5G modules, which can convert various digital information output by the microprocessor 15 into 5G information and transmit the 5G information to the cloud computing center 7 for preventing regulation violation, and convert various received 5G instructions sent from the cloud computing center 7 for preventing regulation violation into digital information which can be received by the microprocessor and input the digital information into the microprocessor. The 5G information converter 5 can also be replaced with wireless or wired information converters such 4G or 6G or Wi-Fi according to actual needs.

The computer or mobile phone terminal 6 can be installed with the violation behavior identification and processing software.

The cloud computing center 7 for preventing regulation violation comprises: a networked computing and processing system such as an Alibaba Cloud platform, an Internet of Things platform, and a scheduling room computer, which can store data and compute and process the data. According to actual needs, a computer system with a computing function, such as a microprocessor or a single-chip microcomputer system, can also be used instead.

The actuator system is any of the electric lock mechanism system 9, a superior power switch sensor 215 for preventing regulation violation, a pipeline electric valve 707, a possible rear-ending vehicle 907, a vehicle power control switch 107, or a mobile phone of an emergency rescuer 807.

The electric lock mechanism system 9 comprises a stepping motor, an electromagnet or other action execution mechanisms as well as control circuits, and an action state of the system is processed by the microprocessor. A logical variable stored in the cloud computing center 7 for preventing regulation violation is represented by H.

The identity identification system 11 comprises a identification facilities for biometric characteristic including voice, image, fingerprint or other biometric characteristic, such as a microphone, a camera, a video camera or other devices, and non-biometric characteristic identification facilities, such as a key, a card reader, a radio frequency card, or a card reader. Full-time operator profile identity information is pre-installed in an database of cloud computing center 7 for preventing regulation violation. Actual identity information of actual operators processed by the microprocessor is also stored in the database of cloud computing center 7 for preventing regulation violation. After comparison computation between the Full-time operator profile identity information and the actual identity information, a logical variable is formed, which is represented by C.

The auxiliary variable E identification system 13 comprises a switch comprehensive protection means that stores voltage, current, short circuit, electric leakage and other electrical parameters, and a radio frequency card reader that reads patrol inspection of an operator. Related state information is processed by the microprocessor and then stored in the database of cloud computing center 7 for preventing regulation violation. A logical variable of the state information is represented by E. An input circuit of the auxiliary variable E identification system 13 comprises a current or voltage load operating state input circuit.

The microprocessor 15 comprises a computer processing system such as a single-chip microcomputer or a PLC. It can achieve wireless output such as 5G/4G/WIFI, bus output, relay contact output, on-site alarm output, and computer or mobile phone terminal 6 output.

The position and environment identification system 17 comprises memories such as an electrically erasable programmable read only memory (EEPROM) on a sensor circuit board, which is configured to store profile information about device performances and sensor positions, and devices such as a temperature probe and a photoresistor probe that are arranged and connected on the intelligent sensor system for preventing regulation violation. Related state information such as environment temperature and environment brightness is sent from the devices to the microprocessor and then processed by the microprocessor, to form a logical variable represented by D, which is stored in the cloud computing center 7 for preventing regulation violation.

The dangerous source information acquisition system 19 comprises a switch comprehensive protection means with a function of acquiring a voltage logical variable, a methane monitoring system and the like. State information of the system is processed by the microprocessor, to form a logical variable represented by X, which is stored in the cloud computing center 7 for preventing regulation violation. Or, after information of a dangerous source including gas, carbon dioxide and the like is directly transmitted to a ground scheduling platform, dangerous source information formed by calling and sharing is directly transmitted to the cloud computing center 7 for preventing regulation violation to form a dangerous source logical variable X.

As shown in Figs. 11a and 11b, a basic workflow of the intelligent sensor system for preventing regulation violation according to some embodiments of the present disclosure comprises: starting; initializing; reading EEPROM configuration information; in case of reading successfully, configuring 5G information converter 5, and connecting the 5G information converter 5 to the cloud computing center 7 for preventing regulation violation; in case of connecting successfully, receiving an command of the cloud computing center for preventing regulation violation; parsing the command and executing the command; and regularly reporting information of dangerous source information acquisition system 19, information of position and environment identification system 17, information of auxiliary variable E identification system 13, information of regulation violation behavior identification system 1 based on lock mechanism, information of regulation violation behavior identification information system 3 based on non-lock mechanism, information of identity identification system 11, and electric lock action information of the electric lock mechanism system 9.

A method of identifying a regulation violation behavior in some embodiments according to the present disclosure comprises: converting dangerous source state information into a value of a variable X by a dangerous source identification system, or converting an operator behavior into a value of a variable B1 by a regulation violation behavior identification system of device-based lock mechanism, or converting an operator behavior into a value of a variable B2 by a regulation violation behavior identification system based on non-lock mechanism arranged in an operating place, or converting identity information of an operator into a value of a variable C by an identity identification system, or converting position information of an operator and surrounding environment information of the operator into a value of a variable D by a position and environment identification system, or converting a state including voltage and current auxiliary variables into a value of a variable E by an auxiliary variable E identification system, or processing action state of an electric lock mechanism system by a microprocessor, to form a value of a logical variable H stored in a cloud computing center for preventing regulation violation, wherein at least one of values of the variables X, B1, B2, C, D, E, or H is formed by being processed by a microprocessor of an intelligent sensor system for preventing regulation violation and then transmitted to a 5G information converter, and then transmitted to the cloud computing center for preventing regulation violation pre-installed with regulation violation behavior identification and processing software. The values of the variables can also be directly called and shared by the cloud computing center for preventing regulation violation from other data systems. The cloud computing center for preventing regulation violation computes the variables X, B1, B2, C, D, E, and H according to an regulation violation behavior identification algorithm, to determine types of regulation violation behavior. The types are transmitted back to the 5G information converter, and then processed by the microprocessor, to command an actuator system to act, for giving an alarm, or cutting off or locking a dangerous source.

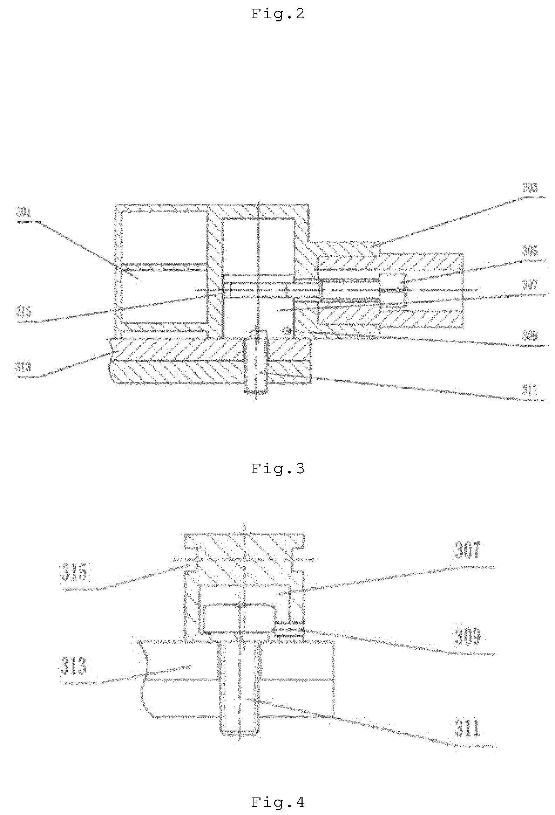

Fig. 2 is a schematic diagram of an identification system for identifying a regulation violation behavior of live operation in an explosive environment according to some embodiments of the present disclosure. As shown in Fig. 2, the identification system for identifying a regulation violation behavior of live operation in an explosive environment in this embodiment comprises: a mining cloud computing center 201 for preventing regulation violation, a load switch sensor 205 for preventing regulation violation, a load 207, a load switch 209, a cable junction box 211, a superior power switch 213, and a superior power switch sensor 215 for preventing regulation violation. The superior power switch sensor 215 for preventing regulation violation is arranged on a junction box of the superior power switch 213. The load switch sensor 205 for preventing regulation violation is arranged on a junction box of the load switch 209 controlling a load 207. The installation method of the sensor as shown in Figs. 3 and 4 comprises: arranging a lossless connection sleeve 307 on a fastening bolt 311 of a housing cover plate 313 of a device; screwing a jackscrew 309 to fix the lossless connection sleeve 307 on the fastening bolt 311; covering a sensor housing 303 ; screwing a lock cylinder 305 into a locking notch 315 of the lossless connection sleeve 307 with a key of the lock cylinder 305 to fix the sensor housing 303 onto the lossless connection sleeve 307. Through the method, the sensor is installed on a cover plate of the switch junction box shown in Fig. 2. The sensor for preventing regulation violation can also be arranged on the cable junction box 211 to implement regulation violation behavior identification, which is specifically implemented as described above.

In a case where a full-time person unscrews the lock cylinder 305 with the special key and removes the sensor housing 303, a microswitch (not shown in the figure) connected with a sensor circuit board 301 is triggered, the microswitch acts to generate a suspected regulation violation signal. The signal is transmitted to the sensor circuit board 301 in Fig. 3, processed by the intelligent sensor system for preventing regulation violation in Fig. 1, and wirelessly transmitted from the 5G information converter 5 to the cloud computing center 7 for preventing regulation violation, to form a suspected regulation violation logical variable B11.

The superior power switch sensor 215 for preventing regulation violation and the load switch sensor 205 for preventing regulation violation are both connected with comprehensive protection 485 communication ports of the superior power switch 213 and the load switch 209. Voltage, current and other electrical parameters of the switches are processed by an interface of the auxiliary variable E identification system 13 in Fig. 1. The parameters after processed are wirelessly transmitted to the cloud computing center 7 for preventing regulation violation by the 5G information converter 5. The cloud computing center 7 for preventing regulation violation preforms "AND" computation on, a power voltage of the load switch 209 represented by XI, and the suspected regulation violation logical variable B11. In a case where $X1*B11=1$, it is determined that the behavior of removing a single load switch sensor 205 for preventing regulation violation, i.e., the behavior of removing the sensor housing 303, is a regulation violation behavior W, which has a logical expression of $W=X1 \times B11$. In a case where $X1=1$ and $B11=1$, $W=I$, which indicates a regulation violation. The mining cloud computing center 201 for preventing regulation violation is suitable for an explosive environment such as a coal mine. The mining cloud computing center 201 for preventing regulation violation comprises computers of an underground substation, a ground scheduling platform, and the like. The mining cloud computing center 201 is a type of the cloud computing center 7 for preventing regulation violation shown in Fig. 1.

The superior power switch sensor 215 for preventing regulation violation is a subsystem of the intelligent sensor system for preventing regulation violation shown in Fig. 1, which differs from the intelligent sensor system in not comprising the cloud computing center 7 for preventing regulation violation, and the computer or mobile phone terminal 6 connected by a dotted line in Fig. 1, but has same other parts such as the microprocessor 15 and the 5G information converter 5 and is installed with software and hardware suitable for the coal mine.

The load switch sensor 205 for preventing regulation violation differs from the superior power switch sensor 215 for preventing regulation violation in an allocated IP address, a network card number, and a function.

As shown in Fig. 5, an identity of a violator can be identified not only by unlocking with the special lock key, but also by a card reader 510 identification system, which has the following identification process: a full-time person uses a special card that is aimed at a card reader 510 for reading the card; a read signal is transmitted from the 5G information converter 5 on the sensor circuit board 301 to the cloud computing center 7 for preventing regulation violation, to form an identity identification logical variable C. After analysis and computation, in a case where the identity is determined to be a full-time person, $C=1$, then an unlocking command is issued to the electric lock mechanism system and transmitted, in a form of a 5G signal, to the load switch sensor 205 for preventing regulation violation in Fig. 2. After received by the 5G information converter 5, the 5G signal is converted into a command signal by the sensor circuit board 301 in Fig. 5, to command the electric lock 517 to move upwards and release locking to open an operation passage of the lock cylinder 521, so that the key 519 can be inserted to operate the lock cylinder 521, and further unlock to remove the load switch sensor 205 for preventing regulation violation in Fig. 2. In a case where it is removed by regulation violation, identity of the violator is identified through the radio frequency card.

An acousto-optic identification probe 509 can also be arranged. After the identity of the operator is identified through the acousto-optic system, the locking of the electric lock 517 can be released, so that the load switch sensor 205 for preventing regulation violation in Fig. 2 can be further removed. In a case where it is removed by regulation violation, identity of the violator is identified through biological characteristics such as voice or image.

The acousto-optic identification probe 509 comprises a microphone, a video camera, and the like. The card reader 510 comprises a radio frequency card reader, a magnetic card reader, and the like. The electric lock 517 comprises an electromagnet, a stepping motor, and the like.

While the above superior power switch 213 transmits power to the load switch 209, live information is transmitted from the superior power switch sensor 215 for preventing regulation violation to the mining cloud computing center 201 for preventing regulation violation and converted into a voltage dangerous source logical variable X1. In a case where there is live, $X1=1$, otherwise, $X1=0$. In a case where $X1=1$, the mining cloud computing center 201 for preventing regulation violation synchronously commands the electric lock of the load switch sensor 205 for preventing regulation violation to be locked, to disable the operator to perform a regulation violation behavior of live uncapping operation, so that a live dangerous source is identified and locked. In a case where X1=0, the mining cloud computing center 201 for preventing regulation violation synchronously commands the electric lock of the load switch sensor 205 for preventing regulation violation to be unlocked, to enable the operator to perform an uncapping operation.

After information of methane and other hazardous gases exceeding a limit (or information on whether there is live) is transmitted to the ground scheduling platform through a methane monitoring system (or an electricity monitoring system), the cloud computing center 7 for preventing regulation violation calls and shares the information of gas exceeding a limit and the information on whether there is live. After receiving a gas-exceeding-limit (or live) logical variable X5=1 (or X1=1), the cloud computing center 7 for preventing regulation violation commands the electric lock 517 to be locked, to ensure that a live operation cannot be performed after the gas exceeds the limit (or live). Note that the information of hazardous gas dangerous source such as methane (or live dangerous source) is not directly transmitted to an interface circuit of the dangerous source information acquisition system 19 shown in Fig. 1, but is obtained by calling and sharing instead of direct transmission.

Embodiment 2.

As shown in Fig. 6, an identification system for identifying a regulation violation behavior of drowsiness based on non-lock mechanism in this embodiment comprises a conveyor magnetic starter 601 and a drowsiness sensor 603 for preventing regulation violation based on non-lock identification. The drowsiness sensor 603 is arranged on the conveyor magnetic starter 601 controlling a conveyor. A switch state signal of the conveyor magnetic starter 601 is transmitted to an interface circuit (shown in Fig. 1) of the dangerous source information acquisition system 19 of the drowsiness sensor 603 through a connecting wire 602, and is transmitted by its 5G information converter 5 to the cloud computing center 7 for preventing regulation violation, acting as conveyor startup or shutdown information, namely dangerous source state information. A logical variable of the information is represented by X2. A contact of an preventing drowsiness means 607 is connected to an input interface circuit of the regulation violation behavior identification system 3 based on non-lock mechanism shown in Fig. 1, to form the identification system for identifying a regulation violation behavior of drowsiness. The information is transmitted to the cloud computing center 7 for preventing regulation violation as a drowsiness logical variable B21. The cloud computing center 7 for preventing regulation violation detects the B21. In a case where the information is not received within a certain time period (such as 5 minutes), let B21=1, and a suspected regulation violation behavior of drowsiness is determined. When on duty daily, a belt conveyor driver 609 is required to press a button of the preventing drowsiness means 607 at regular time such as once every minute. In a case where the button is not pressed for 5 minutes, the driver is considered drowsy. Meanwhile, the belt conveyor driver 609 automatically transmits information such as his own identity to the ground scheduling station through a personnel positioning system 611. The cloud computing center 7 for preventing regulation violation is connected with the scheduling station, and through calling and sharing, takes out the identity information of the belt conveyor driver 609 as identity identification information required by the system, represented by C. In a case where the belt conveyor driver does not press the button of the preventing drowsiness means 607 for 5 minutes, it is indicated that the driver is drowsy, the logical variable in the cloud computing center 7 for preventing regulation violation represented by B21 equals to 1, otherwise B21=0. At this time, if the belt conveyor operates, namely a belt switch is closed, a logical variable in the cloud computing center 7 for preventing regulation violation represented by X2 equals to 1. In a case where W=B21*X2=1, a regulation violation operation is determined, so that the cloud computing center 7 for preventing regulation violation, through the drowsiness sensor 603, commands the conveyor magnetic starter 601 to cut off power to make the conveyor stop operating or to give an alarm, and may also command, at the same time, one previous conveyor or two previous conveyors interlocked with the conveyor to stop operating. The button of the preventing drowsiness means 607 can also be replaced with a sensing switch. In a case where a hand touches a top of the switch, it is equivalent to pressing the button so that a signal is output. In a case where the hand leaves the top of the switch automatically due to drowsiness, it is equivalent to not pressing the button so that the outputting of the signal is terminated. It is also possible to add a logical variable D of a position and environment identification system 17 for the driver, a logical variable C of an identity identification system 11 for the driver, and an auxiliary variable E comprising short circuit, leakage protection and the like of the device operated by the driver of identification system 13, and then perform comparison computation according to the regulation violation behavior identification algorithm.

The drowsiness sensor 603 for preventing regulation violation based on non-lock identification is a subsystem of the intelligent sensor system for preventing regulation violation shown in Fig. 1, which does not comprise the cloud computing center 7 for preventing regulation violation, and the computer or mobile phone terminal 6 connected by a dotted line in Fig.1. The drowsiness sensor 603 has a basically same structure as the superior power switch sensor 215 for preventing regulation violation and the load switch sensor 205 for preventing regulation violation, but uses a different method of identifying the regulation violation behavior.

According to some embodiments of the present disclosure, there is provided an identification system for identifying a regulation violation behavior of not-watching-computer based on non-lock mechanism. The identification system for identifying a regulation violation behavior of not-watching-computer based on non-lock mechanism in the embodiment comprises a probe for preventing regulation violation of not-watching-computer arranged on a computer. The probe may adopt a direct contact switch, which is triggered to signal when sitting down or leaving. Or the probe adopts a photoelectric sensing type device, such as a pair of transmitting and receiving tubes, wherein the transmitting tube continuously emits light, when a human body approaches, the light is reflected, and the receiving tube receives the light, which indicates that a person is watching the computer. if the reflected light is not received, it is indicated that the person watching the computer has left in violation of regulation, without watching the computer. The signal is processed by the sensor, and transmitted via the 5G information converter 5 to the cloud computing center 7 for preventing regulation violation, as a suspected regulation violation logical variable B22. Depending on a post, an allowed continuous not-watching-computer time T is stipulated. If a time is greater than T, a suspected regulation violation is determined. For example, if the allowed continuous not-watching-computer time T=5 minutes is stipulated, in a case where a continuous not-watching-computer time is 6 minutes, a suspected regulation violation is determined. In practical applications, the allowed continuous not-watching-computer time T is stored in the cloud computing center 7 for preventing regulation violation. In a case where the continuous not-watching-computer time is greater than T, let B22=1, otherwise B22=0.

A device startup/shutdown state data system monitored by the computer is networked with the cloud computing center 7 for preventing regulation violation for calling and sharing data. Startup/shutdown dangerous source state information is converted into a value of a logical variable X. In a case where it is a startup state, X3=1, otherwise X3=0. Therefore, in a case where B22*X3=1, a regulation violation behavior of off-duty is determined, so that an alarm is given or a workflow controlled by the computer is stopped to prevent an accident. The means can also be changed into a means of identifying a regulation violation behavior of watching-computer of a child, which is omitted here.

According to some embodiments of the present disclosure, there is provided an identification system for identifying a regulation violation behavior of hazardous gas leakage. As shown in Fig. 7, the identification system for identifying a regulation violation behavior of hazardous gas leakage in this embodiment comprises a manual valve leakage sensor 701 for preventing regulation violation based on non-lock identification, a manual valve 703, a hazardous gas pipeline 705, a pipeline electric valve 707, and an electric valve leakage sensor 709 for preventing regulation violation based on non-lock identification. The manual valve leakage sensor 701 for preventing regulation violation based on non-lock identification is arranged beside the manual valve 703 of the hazardous gas pipeline 705. A dangerous source information acquisition system of the manual valve leakage sensor 701 for preventing regulation violation based on non-lock identification is connected with a hazardous gas probe. The electric valve leakage sensor 709 for preventing regulation violation based on non-lock identification is arranged beside the pipeline electric valve 707 of the hazardous gas pipeline 705. In a case where it is measured by the hazardous gas probe that the hazardous gas exceeds a specified value, the information is processed by the microprocessor 15 and sent from the 5G information converter 5 to the cloud computing center 7 for preventing regulation violation to form a hazardous gas dangerous source logical variable X4. In a case where the hazardous gas measured exceeds a specified value, X4=1, otherwise, X4=0. In a case where hazardous gas leakage is caused by regulation violation, the manual valve leakage sensor 701 for preventing regulation violation based on non-lock identification wirelessly reports information of whether there is an operator on site to the cloud computing center 7 for preventing regulation violation while giving an alarm on site, to form a logical variable B23 through computation. In a case where there is an operator, B23=1, otherwise B23=0. In a case where B23*X4=1, a regulation violation is determined, so that the electric valve leakage sensor 709 for preventing regulation violation based on non-lock identification is commanded to issue an instruction to cause the pipeline electric valve 707 to act to close the hazardous gas pipeline, and meanwhile, to turn on ventilation facilities such as electric fans on site, and to notify a related person for emergency treatment through a mobile phone.

The manual valve leakage sensor 701 for preventing regulation violation based on non-lock identification is a subsystem of the intelligent sensor system for preventing regulation violation shown in Fig.1. The manual valve leakage sensor 701 does not comprise the cloud computing center 7 for preventing regulation violation, and the computer or mobile phone terminal 6 connected by a dotted line in Fig.1.

The electric valve leakage sensor 705 for preventing regulation violation based on non-lock identification differs from the manual valve leakage sensor 701 for preventing regulation violation based on non-lock identification in an allocated IP address, a network card number, and a function.

The hazardous gas includes: civil petroleum, natural gas, liquefied gas, etc.

Methane, which is a main hazardous gas underground, can be measured by a set of mine methane monitoring systems. A measured concentration value of methane dangerous source is directly sent to a ground scheduling station. The cloud computing center 7 for preventing regulation violation is networked with the ground scheduling station for calling and sharing the concentration value data of methane dangerous source. A methane dangerous source logical variable X5 is formed through comparison computation. In a case where the value exceeds a limit, x5=1, otherwise X5=0. Similarly, in a case where B23*X5=1, a regulation violation behavior of entry to a methane area is determined, so that a related person is commanded to evacuate and an alarm is given.

According to some embodiments of the present disclosure, there is provided an identification system for identifying a regulation violation behavior of entering a dangerous area. As shown in Fig. 8, the identification system for identifying a regulation violation behavior of entering a dangerous area in this embodiment comprises an entry sensor 811 for preventing regulation violation based on non-lock identification and a regulation violation red line 803, wherein: the regulation violation red line 803 is provided at an entrance to the dangerous area; the entry sensor 811 for preventing regulation violation based on non-lock identification is arranged on the regulation violation red line 803; and a human body sensing probe is arranged on the entry sensor 811 for preventing regulation violation based on non-lock identification. The entry sensor 811 for preventing regulation violation based on non-lock identification continuously sends information of its position, acting as regulation violation red line marker information, to the cloud computing center 7 for preventing regulation violation, to form a logical variable X6. In a case where the regulation violation red line marker information is received, X6=1, otherwise X6=0. In a case where an operator 809 in a dangerous area is sensed by the human body sensing probe, which is equivalent to touching the regulation violation red line 803, the entry sensor 811 for preventing regulation violation based on non-lock identification gives an alarm on site before the operator enters a dangerous area 801, and reports information through its 5G information converter 5 to the cloud computing center 7 for preventing regulation violation to form a logical variable B24. In a case where the regulation violation red line is touched, B24=1, otherwise, B24=0. In a case where B24*X6=1, a regulation violation is determined, so that a command is issued to a mobile phone of an emergency rescuer 807 in an underground safe operating area 805 by the cloud computing center 7 for preventing regulation violation, to prevent the regulation violation behavior of the operator 809 in dangerous area as soon as possible.

The entry sensor 811 for preventing regulation violation based on non-lock identification is a subsystem of the intelligent sensor system for preventing regulation violation shown in Fig. 1. The entry sensor 811 does not comprise the cloud computing center 7 for preventing regulation violation, and the computer or mobile phone terminal 6 connected by a dotted line in Fig. 1.

The regulation violation red line 803 is a door, railing, or other marker with a no-admittance prompt.

The dangerous area is an underground methane area with high concentration where is prohibited from entering, an underground pipeline with insufficient oxygen, a nuclear pollution area, a hospital X-ray radiation area, or the like.

According to some embodiments of the present disclosure, there is provided an identification system for identifying a violator based on an unmanned vehicle. As shown in Fig. 9, the system for identifying a violator based on an unmanned vehicle in this embodiment comprises a dangerous source speed probe and a crash sensor 900 for preventing regulation violation based on non-lock identification. The dangerous source speed probe and the crash sensor 900 for preventing regulation violation based on non-lock identification are arranged on an unmanned vehicle 905 and a possible rear-ending vehicle 907. Speed values of the unmanned vehicle 905 and the possible rear-ending vehicle 907 driving on a road 901 that are measured by the dangerous source speed probes of the vehicles are directly inputted into an interface circuit of a dangerous source information acquisition system of the crash sensor 900 for preventing regulation violation based on non-lock identification, processed by the microprocessor 15 and sent via the 5G information converter 5 to the cloud computing center 7 for preventing regulation violation, to form a logical variable X7 of moving vehicle dangerous source. In a case where the speed reaches a danger value, X7=1, otherwise, X7=0. A human body sensing probe such as radar is arranged on the crash sensor 900 for preventing regulation violation based on non-lock identification. In a case where a pedestrian 903 of regulation violation entry to the road is detected, the human body sensing probe transmits related data through the 5G information converter 5 to the cloud computing center 7 for preventing regulation violation to form a logical variable B25. The cloud computing center 7 for preventing regulation violation compares a distance between the pedestrian and the vehicle with an originally set dangerous distance value. In a case where a distance between the pedestrian and the vehicle approaches the set value, B25=1, otherwise B25=0. In a case where the distance is less than or equal to the dangerous distance value, namely: B25*X7=1, the unmanned vehicle 905 and the possible rear-ending car 907 are commanded to brake to ensure personnel safety. A personnel position can also be found through BeiDou Navigation System, GPS Navigation System, or a underground personnel positioning system, which all need to be connected with the cloud computing center 7 for preventing regulation violation and perform data calling and sharing with the cloud computing center 7 for preventing regulation violation. The crash sensor 900 for preventing regulation violation based on non-lock identification uninterruptedly sends data to the cloud computing center 7 for preventing regulation violation or receives data therefrom. In a case where a violator is found, the vehicle is commanded to stop and an alarm is given.

The crash sensor 900 for preventing regulation violation based on non-lock identification is a subsystem of the intelligent sensor system for preventing regulation violation shown in Fig. 1. The crash sensor 900 does not comprise the cloud computing center 7 for preventing regulation violation, and the computer or mobile phone terminal 6 connected by a dotted line in Fig. 1.

The unmanned vehicle can also be a common vehicle or an underground rubber-tired vehicle.

According to some embodiments of the present disclosure, there is provided an identification system for identifying a regulation violation behavior of entering a rail transport lane where a pedestrian is not admitted. As shown in Fig. 10, the identification system for identifying a regulation violation behavior of entering a rail transit lane where a pedestrian is not admitted in this embodiment comprises a pedestrian sensor 105 for preventing regulation violation based on non-lock identification, a vehicle power control switch 107, and a laser 109, wherein: the pedestrian sensor 105 for preventing regulation violation based on non-lock identification is arranged and connected on the vehicle power control switch 107; the laser 109 is arranged beside a rail 101 of the rail transport lane; and the pedestrian sensor 105 for preventing regulation violation based on non-lock identification is connected with the laser 109. Upon a person 100 violates regulation to enter the rail 101, laser emitted by the laser is shielded, the information is sent to the cloud computing center 7 for preventing regulation violation by a 5G information converter 5 corresponding to the pedestrian sensor 105 for preventing regulation violation based on non-lock identification, as a logical variable B26 for suspected regulation violation behavior of entry, and let B26=1. An on/off state signal of the vehicle power control switch 107 is transmitted to the cloud computing center 7 for preventing regulation violation, as a dangerous source logical variable X8. In a case where the switch is on, it is indicated that a rail transport vehicle 103 is in danger of crashing into the person during moving, at this time, X8=1. In a case where B26*X8=1, by performing comparison computation with identity identification information C and environment information D, it can be specifically determined that a certain person has the regulation violation behavior of entry at a certain place. And meanwhile, the vehicle power control switch 107 is commanded to cut off power and give an alarm, to ensure safety of the person. Since the identity and the place can be accurately identified, the violator can also be automatically fined. This embodiment can also be used for identifying behaviors such as a regulation violation behavior of belt crawling. The laser can also be replaced with a common stay wire. In a case where an operator touches the stay wire, the switch acts to signal.

The pedestrian sensor 105 for preventing regulation violation based on non-lock identification is a subsystem of the intelligent sensor system for preventing regulation violation shown in Fig. 1. The pedestrian sensor 105 does not comprise the cloud computing center 7 for preventing regulation violation, and the computer or mobile phone terminal 6 connected by a dotted line in Fig. 1.

The laser 109 comprises a transmitter and a receiver.

The rail 101 of the rail transport lane is a winch transport rail, an electric locomotive transport rail or a high-speed train rail.

The rail transport vehicle 103 is an overhead line electric locomotive, a winch hoist, or a high-speed train.

According to some embodiments of the present disclosure, there is provided an identification system for identifying a regulation violation behavior of live operation in a household or a workshop. The identification system for identifying a regulation violation behavior of live operation in a household or a workshop in this embodiment comprises a human body sensing probe with a wireless alarm function which is arranged on civil electricity or electric equipment in a production workshop. In a case where a human body approaches a live object, an alarm is given from the human body sensing probe to an intelligent sensor for preventing regulation violation arranged beside a main switch. The intelligent sensor for preventing regulation violation commands to cut off power. And if current level power cannot be cut off for some reason, the intelligent sensor can commands to cut off its superior level power.

Fig. 12 is a schematic flow diagram of a method of preventing regulation violation behavior according to some embodiments of the present disclosure. As shown in Fig. 12, the method of preventing regulation violation behavior of the embodiment of the present disclosure comprises step S1210 to step S1230.

In step S1210, dangerous source state information and behavior information of an operator are obtained.

In some embodiments, the regulation violation behavior comprises at least one of: a regulation violation behavior of uncovering operation in an explosive environment, drowsy driving, a regulation violation behavior of off-duty, a regulation violation behavior of hazardous gas leakage, or a regulation violation behavior of entry to a dangerous area.

In some embodiments, in a case where the regulation violation behavior comprises uncapping operation in the explosive environment, step S1210 comprises at least one of: obtaining dangerous source state information of whether a target device is live and behavior information of whether the operator performs uncapping on the target device; or obtaining dangerous source state information of whether a hazardous gas in an operation environment exceeds a limit threshold and behavior information of whether the operator performs uncapping on the target device.

In step S1220, whether the operator has a regulation violation behavior is determined, according to the dangerous source state information and the behavior information of the operator.

In some embodiments, in a case where the regulation violation behavior comprises the uncapping operation in the explosive environment, step S1220 comprises: in a case where the dangerous source state information indicates that the target device is live or the hazardous gas in the operation environment exceeds the limit threshold and the behavior information indicates that the operator performs uncapping on the target device, determining that the operator has the regulation violation behavior of uncapping operation in the explosive environment; otherwise, determining that the operator does not have the regulation violation behavior of the uncapping operation in the explosive environment.

In step S1230, in a case where the operator has the regulation violation behavior, an actuator system is commanded to execute a first action.

The first action is used for preventing the regulation violation behavior.

In some embodiments, the method of preventing regulation violation behavior further comprises: obtaining identity information of the operator; in a case where the operator has the regulation violation behavior, determining an identity of a violator according to the identity information of the operator; and in a case where the operator does not have the regulation violation behavior and identity authentication performed according to the identity information of the operator is passed, commanding the actuator system to execute a second action, wherein the second action is used for allowing the operation behavior.

In the embodiment of the present disclosure, the regulation violation behavior can be accurately identified by the above method, and the regulation violation behavior can be timely processed, so that efficiency and accuracy of governance of the regulation violation behavior are improved.

Fig. 13 is a schematic structural diagram of an apparatus of preventing regulation violation behavior according to some embodiments of the present disclosure. As shown in Fig. 13, an apparatus 1300 of preventing regulation violation behavior of the embodiment of the present disclosure comprises: an obtaining module 1310, a determining module 1320, and a control module 1330.

The obtaining module 1310 is configured to obtain dangerous source state information and behavior information of an operator.

The determining module 1320 is configured to, according to the dangerous source state information and the behavior information of the operator, determine whether the operator has a regulation violation behavior.

The control module 1330 is configured to, in a case where the operator has the regulation violation behavior, command an actuator system to perform a first action, wherein the first action is used for preventing the regulation violation behavior.

In the embodiment of the present disclosure, the regulation violation behavior can be accurately identified by the above apparatus, and the regulation violation behavior can be timely processed, so that efficiency and accuracy of governance of the regulation violation behavior are improved.

Fig. 14 is a schematic structural diagram of a system of preventing regulation violation behavior according to some embodiments of the present disclosure. As shown in Fig. 14, the system 1400 of preventing regulation violation behavior of the embodiment of the present disclosure comprises an acquisition device 1410 and a cloud computing center 1420.

The acquisition device 1410 is configured to acquire dangerous source state information and behavior information of an operator.

In some embodiments, the regulation violation behavior comprises at least one of: a regulation violation behavior of uncapping operation in an explosive environment, drowsy driving, a regulation violation behavior of off-duty, a regulation violation behavior of hazardous gas leakage, or a regulation violation behavior of entry to a dangerous area.

In some embodiments, in a case where the regulation violation behavior comprises the uncapping operation in the explosive environment, the acquisition device comprises: at least one of a first dangerous source state information acquisition device or a second dangerous source state information acquisition device, and a behavior information acquisition device.

The first dangerous source state information acquisition device is configured to acquire dangerous source state information of whether a target device is live.

The second dangerous source state information acquisition device is configured to acquire dangerous source state information of whether a hazardous gas in an operation environment exceeds a limit threshold.

In some embodiments, the second dangerous source state information acquisition device comprises: a hazardous gas probe; and a leakage sensor for preventing regulation violation configured to, in a case where the hazardous gas probe detects that a gas concentration exceeds the limit threshold, send the state information of hazardous gas dangerous source to the cloud computing center 1420.

The behavior information acquisition device is configured to acquire behavior information of whether the operator performs uncapping on the target device.

In some embodiments, the behavior information acquisition device is shown in Fig. 3, comprising: a sensor housing 303; a lock cylinder 305, which is arranged in a hole provided on one sidewall of the sensor housing 303 and configured to fix the sensor housing 303 onto a housing cover plate 313 of a device; a sensor circuit board 301, which is provided inside the sensor housing 303, in signal connection with a microswitch node or a Hall element active switch, and configured to, after detecting behavior information that the operator triggers the microswitch due to removing the lock cylinder and the sensor housing, send the behavior information to the cloud computing center.

In some embodiments, the behavior information acquisition device further comprises: a lossless connection sleeve 307, which is fixedly arranged on a bolt head of a fastening bolt 311 of the housing cover plate 313 of the device through a jackscrew 309; and a sensor housing 303, which is covered onto the lossless connection sleeve 307 and fixed onto the lossless connection sleeve 307 by screwing a lock cylinder 305 into a locking notch 315 of the lossless connection sleeve 307 with a key corresponding to the lock cylinder 305.

In other embodiments, the behavior information acquisition device is as shown in Figs. 3 and 5, comprising: a sensor housing 303, which is fixedly arranged on a device cover plate 525 and connected with a lock sleeve 527 to form an inner cavity of the sensor housing 303; an electric lock 517, which is arranged on the lock sleeve 527 and configured to, after identity identification for an operator performed by the cloud computing center 1420 is passed, act according to an instruction issued by the cloud computing center, to enable a key 519 to operate a lock cylinder 521 for unlocking to remove the sensor housing 303; and a sensor circuit board 301, which is arranged on the inner cavity of the sensor housing 303 and configured to, in a case where the sensor housing 303 is removed, send behavior information to the cloud computing center 1420.

In other embodiments, the behavior information acquisition device further comprises an acousto-optic identification probe 509 and a card reader 510, which are connected with a sensor circuit board 301. The sensor circuit board 301 is further configured to send, to the cloud computing center 1420, voice or image information of the operator obtained through the acoustic-optical identification probe 509 or personal code information of a radio frequency card or a magnetic card obtained through the card reader 510, wherein the voice or image information of the operator or the personal code information is used for identification of the operator.

In other embodiments, the behavior information acquisition device further comprises: a lossless connection cover 523, which is fastened onto a device cover plate 525 through a lossless connection nut 512 and a lossless connection bolt 513; a lossless connection bolt sleeve 515, which is sleeved onto the lossless connection bolt 513. The lock sleeve 527 is arranged on the lossless connection bolt sleeve 515 and fixed on the lossless connection bolt sleeve 515 through a lossless connection lock cylinder 521.

The cloud computing center 1420 is configured to: receive the dangerous source state information and the behavior information of the operator sent from the acquisition device; determine whether the operator has the regulation violation behavior according to the dangerous source state information and the behavior information of the operator; and in a case where the operator has the regulation violation behavior, command an actuator system to execute a first action, for preventing the regulation violation behavior.

In the embodiment of the present disclosure, the regulation violation behavior can be accurately identified by the above system, and the regulation violation behavior can be timely processed, so that efficiency and accuracy of governance of the regulation violation behavior are improved.

The beneficial effects of the embodiment of the present disclosure are as follows.

Since a sensor is a device for converting a non-electrical physical quantity such as temperature and pressure into an electrical physical quantity, temperature sensor, pressure sensor and other sensors concentrate on materials. In a case where temperature or pressure changes, a value of change in a physical parameter of a material caused by this is taken out and processed into electrical information for monitoring system, which is the manner that a general sensor function is achieved. According to this idea, it is difficult or even almost impossible to find a change in a physical parameter of a certain material caused by a regulation violation behavior, so that the application of the sensor to identify the regulation violation behavior becomes a worldwide difficulty. In this disclosure, by innovatively combining common elements such as existing biguardian-lock mechanism, button, voltage sensor, methane probe, and network system, a new intelligent sensor system for preventing regulation violation (note that it is not a sensor) is formed. The system performs computation and analysis according to the regulation violation behavior identification algorithm of this disclosure, and determines whether it is a regulation violation behavior according to the computation result. The intelligent sensor system for preventing regulation violation has outstanding system functions, and has substantive progress compared with traditional sensors such as temperature sensor and pressure sensor which are designed and manufactured based on a certain element sensing parameter. Therefore this disclosure starts a new idea of developing an intelligent sensor. Compared with the patent of "PRE-COVER-OPENING POWER-OFF METHOD AND APPARATUS" in the related art, cloud computing process such as power voltage computation, violator identity identification, and environmental parameters computation are added, so that the determination of the regulation violation behavior is substantively improved. Compared with the traditional sensors such as temperature sensor and pressure sensor which are designed and manufactured based on a certain element sensing parameter, the cloud computing process such as violator identity identification and environmental parameters computation are added, so that precision of the regulation violation behavior identification is greatly improved.

By using the cloud computing technology, it is possible to give an alarm to tens, hundreds, and thousands of meters away and cut off dangerous source information without laying communication wires, to lock a faraway dangerous source such that it cannot supply power to a regulation violation place or send out a flammable and explosive gas. This technical function can solve the century difficulty of cutting off power for locking during maintenance of a high-voltage line with a long power supply distance, instead of using an old method of short-circuiting the high-voltage line and grounding for locking during maintenance.

The regulation violation behavior identification algorithm of the intelligent sensor system for preventing regulation violation in this disclosure can be applied to various sensor systems, to conveniently identify any regulation violation behavior and violator identity, thereby pre-controlling the regulation violation behavior, completely eradicating regulation violation operations.

According to the present disclosure, computation is performed in the preventing regulation violation cloud computing center using 5G wireless communication technologies (including 4G/Wi-Fi and the like), which has a great significance that difficulty in programming and processing of a single-chip microcomputer on site is greatly reduced, and comparison computation can be performed, in the cloud computing center for preventing regulation violation, for data of different systems at thousands of kilometers away, so that the intelligent sensor system for preventing regulation violation of this disclosure has a very remarkable regulation violation behavior identification and computation function compared with a common single sensor. The cloud computing center for preventing regulation violation can, by a data calling and sharing technology, call technical data of an operator during operation monitored under different systems (such as a methane monitoring system, a personnel positioning system, an electricity monitoring system, and a video monitoring system) to the cloud computing center at a high speed of 5G, perform computation and analysis by using a regulation violation behavior identification algorithm, to identify any regulation violation behavior, thereby thoroughly solving the difficulty of regulation violation behavior identification, further eradicating the regulation violation operation, and realizing an ideal target of reducing an accident occurrence probability by 90%.

According to some embodiments of the present disclosure, there is further provided another apparatus for preventing regulation violation behavior, comprising: a memory; and a processor coupled to the memory, the processor being configured to perform, based on instructions stored in the memory, the method of preventing regulation violation behavior as described above.

According to some embodiments of the present disclosure, there is further provided a computer-readable storage medium having stored thereon computer program instructions which, when executed by a processor, implement the method of preventing regulation violation behavior as described above.

According to some embodiments of the present disclosure, there is further provided a computer program, comprising: instructions which, when executed by a processor, cause the processor to perform the method of preventing regulation violation behavior as described above.

The present disclosure may be implemented in various forms without departing from the spirit and scope of the present disclosure, and it should be understood that the above embodiments are not limited by the foregoing details, but rather should be construed broadly within the scope defined by the claims. It should be noted that, for one of ordinary skill in the art, several modifications and variations within the equivalent scope can also be made without departing from the structure of the present disclosure, and these modifications and variations should also be considered as the scope of protection of the present disclosure.

**Claims**

1. A method of preventing regulation violation behavior, comprising:

   obtaining dangerous source state information and behavior information of an operator;
   determining whether the operator has a regulation violation behavior according to the dangerous source state information and the behavior information of the operator; and
   in a case where the operator has the regulation violation behavior, commanding an actuator system to execute a first action for preventing the regulation violation behavior.

2. The method of preventing regulation violation behavior according to claim 1, wherein the regulation violation behavior comprises at least one of: a regulation violation behavior of uncapping operation in an explosive environment, drowsy driving, a regulation violation behavior of off-duty, a regulation violation behavior of hazardous gas leakage, or a regulation violation behavior of entry to a dangerous area.

3. The method of preventing regulation violation behavior according to claim 2, wherein in a case where the regulation violation behavior comprises the regulation violation behavior of uncapping operation in the explosive environment, the obtaining dangerous source state information and behavior information of an operator comprises at least one of:

   obtaining dangerous source state information of whether a target device is live and behavior information of whether the operator performs uncapping on the target device; or
   obtaining dangerous source state information of whether hazardous gas in an operation environment exceeds a limit threshold and behavior information of whether the operator performs uncapping on the target device.

4. The method of preventing regulation violation behavior according to claim 3, wherein the determining whether the operator has a regulation violation behavior according to the dangerous source state information and the behavior information of the operator comprises:

in a case where the dangerous source state information indicates that the target device is live or the hazardous gas in the operation environment exceeds the limit threshold, and the behavior information indicates that the operator performs uncapping on the target device, determining that the operator has the regulation violation behavior of the uncapping operation in the explosive environment; otherwise, determining that the operator does not have the regulation violation behavior of the uncapping operation in the explosive environment.

5. The method of preventing regulation violation behavior according to any of claims 1 to 4, further comprising:

obtaining identity information of the operator;
in a case where the operator has the regulation violation behavior, determining an identity of a violator according to the identity information of the operator; and
in a case where the operator does not have the regulation violation behavior and identity authentication performed according to the identity information of the operator is passed, commanding the actuator system to execute a second action for allowing the operation behavior.

6. An apparatus of preventing regulation violation behavior, comprising:

an obtaining module configured to obtain dangerous source state information and behavior information of an operator;
a determining module configured to determine whether the operator has a regulation violation behavior according to the dangerous source state information and the behavior information of the operator; and
a control module configured to, in a case where the operator has the regulation violation behavior, command an actuator system to execute a first action for preventing the regulation violation behavior.

7. A system of preventing regulation violation behavior, comprising:

an acquisition device configured to acquire dangerous source state information and behavior information of an operator; and
a cloud computing center configured to:

receive the dangerous source state information and the behavior information of the operator sent from the acquisition device;
determine whether the operator has a regulation violation behavior according to the dangerous source state information and the behavior information of the operator; and
in a case where the operator has the regulation violation behavior, command an actuator system to execute a first action for preventing the regulation violation behavior.

8. The system of preventing regulation violation behavior according to 7, wherein the regulation violation behavior comprises at least one of: a regulation violation behavior of uncapping operation in an explosive environment, drowsy driving, a regulation violation behavior of off-duty, a regulation violation behavior of hazardous gas leakage, or a regulation violation behavior of entry to a dangerous area.

9. The system of preventing regulation violation behavior according to 8, wherein in a case where the regulation violation behavior comprises the regulation violation behavior of uncapping operation in the explosive environment, the acquisition device comprises: at least one of a first dangerous source state information acquisition device or a second dangerous source state information acquisition device, and an behavior information acquisition device,

wherein: the first dangerous source state information acquisition device is configured to acquire dangerous source state information of whether a target device is live;
the second dangerous source state information acquisition device is configured to acquire dangerous source state information of whether hazardous gas in an operation environment exceeds a limit threshold; and
the behavior information acquisition device is configured to acquire behavior information of whether the operator performs uncapping on the target device.

10. The system of preventing regulation violation behavior according to 9, wherein the behavior information acquisition device comprises:

a sensor housing (303);
a lock cylinder (305), which is arranged in a hole provided on one sidewall of the sensor housing (303) and fixes the sensor housing (303) onto a housing cover plate (313) of a device;
a sensor circuit board (301), which is provided inside the sensor housing (303), in signal connection with a microswitch node or a Hall element active switch, and configured to, after detecting behavior information that the operator triggers the microswitch due to removing the lock cylinder and the sensor housing, send the behavior information to the cloud computing center.

11. The system of preventing regulation violation behavior according to 10, wherein the behavior information acquisition device further comprises:
a lossless connection sleeve (307), which is fixedly arranged on a bolt head of a fastening bolt (311) of the housing cover plate (313) of the device through a jackscrew (309), and configured to cooperate with the lock cylinder (305) to fix the sensor housing (303) onto the housing cover plate (313) of the device.

12. The system of preventing regulation violation behavior according to 9, wherein the behavior information acquisition device comprises:

a sensor housing (303), which is fixedly arranged on a device cover plate (525) and connected with a lock sleeve (527) to form an inner cavity of the sensor housing (303);
an electric lock (517), which is arranged on the lock sleeve (527) and configured to, after identity identification performed for the operator by the cloud computing center is passed, act according to an instruction issued by the cloud computing center, to enable a key (519) to operate a lock cylinder (521) for unlocking to remove the sensor housing (303); and
a sensor circuit board (301), which is arranged on the inner cavity of the sensor housing (303), and configured to, in a case where the sensor housing (303) is removed, send the behavior information to the cloud computing center.

13. The system of preventing regulation violation behavior according to 12, wherein:

the behavior information acquisition device further comprises an acousto-optic identification probe (509) and a card reader (510), which are connected with the sensor circuit board (301); and
the sensor circuit board (301) is further configured to send, to the cloud computing center, voice or image information of the operator obtained through the acousto-optic identification probe (509) or personal code information of a radio frequency card or a magnetic card obtained through the card reader (510), wherein the voice or image information of the operator or the personal code information is used for performing identity identification on the operator.

14. The system of preventing regulation violation behavior

ior according to 12, wherein the behavior information acquisition device further comprises:

a lossless connection cover (523), which is fastened onto a device cover plate (525) through a lossless connection nut (512) and a lossless connection bolt (513); and
a lossless connection bolt sleeve (515), which is sleeved onto the lossless connection bolt (513),
the lock sleeve (527) being arranged on the lossless connection bolt sleeve (515) and fixed on the lossless connection bolt sleeve (515) through a lossless connection lock cylinder (521).

15. The system of preventing regulation violation behavior according to 9, wherein the second dangerous source state information acquisition device comprises:

a hazardous gas probe; and
a leakage sensor for preventing regulation violation, which is configured to, in a case where the hazardous gas probe detects that concentration of the hazardous gas exceeds the limit threshold, send the dangerous source state information of the hazardous gas to the cloud computing center.

16. An apparatus of preventing regulation violation behavior, comprising:

a memory; and
a processor coupled to the memory, the processor being configured to perform, based on instructions stored in the memory, the method of preventing regulation violation behavior according to any of claims 1 to 5.

17. A computer-readable storage medium having thereon stored computer program instructions which, when executed by a processor, implement the method of preventing regulation violation behavior according to any of claims 1 to 5.

18. A computer program, comprising:
instructions which, when executed by a processor, cause the processor to perform the method of preventing regulation violation behavior according to any of claims 1 to 5.

19. A method of identifying a regulation violation behavior, comprising:

converting dangerous source state information into a value of a variable X by a dangerous source identification system,

or converting behavior of an operator into a value of a variable B1 by a regulation violation behavior identification system of device-based lock mechanism,

or converting behavior of the operator into a value of a variable B2 by a regulation violation behavior identification system based on non-lock mechanism arranged in an operating place,

or converting identity information of the operator into a value of a variable C by an identity identification system,

or converting information of position of the operator and information of a surrounding environment of the operator into a value of a variable D by a position and environment identification system,

or converting a state comprising voltage and current auxiliary variables into a value of a variable E by an auxiliary variable E identification system, processing action state of an electric lock mechanism system by a microprocessor and storing a value of a logical variable H in a cloud computing center for preventing regulation violation, wherein at least one of values of the variables X, B1, B2, C, D, E, or H is formed by being processed by a microprocessor of an intelligent sensor system for preventing regulation violation and then transmitted to a 5G information converter and then transmitted to the cloud computing center for preventing regulation violation preinstalled with identification and processing software for preventing regulation violation behavior, or the values of the variables are directly called and shared by the cloud computing center for preventing regulation violation from other data system;

determining types of regulation violation behavior, by the cloud computing center for preventing regulation violation, through computing the variables X, B1, B2, C, D, E, and H according to an identification algorithm of preventing regulation violation behavior; and

transmitting the types back to the 5G information converter;

processing the types by the microprocessor for commanding an actuator system to act, giving an alarm, cutting off a dangerous source or locking the dangerous source.

20. The method of identifying a regulation violation behavior according to claim 19, wherein the dangerous source identification system comprises a dangerous source identification and locking system for a power supply system, an identification system for identifying a drowsiness dangerous source upon a regulation violation behavior of drowsiness, an identification system for identifying a not-watching-computer dangerous source upon a regulation violation behavior of not-watching-computer, an identification system for identifying hazardous gas dangerous source, an identification system for hazardous gas methane dangerous source, an identification system for a regulation violation behavior of entering a dangerous area, an identification system for identifying unmanned-vehicle (905) dangerous source, and an identification system for identifying a regulation violation behavior of entering a rail transport lane where a pedestrian is not admitted;

the dangerous source identification and locking system for the power supply system comprises a feed sensor based on the power supply system, or a photoelectric voltage converter of a breaker, or a voltage sensor, or a voltage analog-to-digital converter circuit for a monitoring system, or a voltage dangerous source identification system for a switch comprehensive protection means, wherein: the dangerous source identification and locking system converts dangerous voltage of supply power into safe voltage through a voltage conversion circuit; the safe voltage is transmitted to the microprocessor through a port of a dangerous source information acquisition system of the intelligent sensor system for preventing regulation violation, processed by the microprocessor, and then sent from the 5G information converter to the cloud computing center for preventing regulation violation, to form a voltage dangerous source logical variable X1; in a case where there is dangerous voltage, X1=1, otherwise X1=0;

the identification system for identifying a drowsiness dangerous source upon a regulation violation behavior of drowsiness comprises a conveyor magnetic starter (601), a drowsiness sensor (603) for preventing regulation violation behavior based on non-lock identification, and the cloud computing center (7) for preventing regulation violation, wherein: the drowsiness sensor (603) is arranged on the conveyor magnetic starter (601) controlling a conveyor; a switch state signal of the conveyor magnetic starter (601)is transmitted to the drowsiness sensor (603) through a connecting wire (602); drowsiness sensor (603) transmits the switch state signal for controlling startup or shutdown of the conveyor by the conveyor magnetic starter (601), which is acting as dangerous source state information of the system, to the microprocessor (15) through a port of a dangerous source information acquisition system of the intelligent sensor system for preventing regulation violation; the switch state signal is processed by the microprocessor (15) and then transmitted from a 5G information converter (5) to the cloud computing center (7) for preventing regulation violation, to form a drowsiness dangerous source logical variable X2; in a case where the switch state signal is startup, X2=1; in a case where the switch state signal is shutdown, X2=0;

the identification system for identifying a not-watching-computer dangerous source upon a regulation violation behavior of not-watching-computer comprises a device startup/shutdown state data system monitored by a computer and the cloud computing center (7) for preventing regulation violation, wherein: the system is networked with the cloud computing center (7) for calling and sharing the startup/shutdown dangerous source state information; the startup/shutdown dangerous source state information is sent to the cloud computing center (7), and then converted into a logical variable X3; in a case where the dangerous source state information is startup, X3=1, otherwise X3=0;

the hazardous gas dangerous source identification system comprises a hazardous gas probe, and a manual valve leakage sensor (701)for preventing regulation violation based on non-lock identification connected therewith, wherein: in a case where a concentration value measured by the hazardous gas probe exceeds a specified value, the concentration value is transmitted to the microprocessor (15) through a port of a dangerous source information acquisition system of the intelligent sensor system for preventing regulation violation, processed by the microprocessor (15), and then sent from the 5G information converter (5) to the cloud computing center (7)for preventing regulation violation, to form a hazardous gas dangerous source logical variable X4; in a case where the concentration value exceeds the specified value, X4=1, otherwise X4=0;

the identification system for identifying hazardous gas methane dangerous source comprises a mine methane monitoring system, wherein: the mine methane monitoring system is networked with the cloud computing center (7) for preventing regulation violation through a ground scheduling station; a measured methane dangerous source concentration value is directly sent to the ground scheduling station; the cloud computing center (7) for preventing regulation violation is networked with the ground scheduling station for calling and sharing the methane dangerous source concentration value;and the cloud computing center (7) for preventing regulation violation is configured to form a methane dangerous source logical variable X5 through comparison computation; in a case where the methane dangerous source concentration value exceeds a limit: X5=1, otherwise X5=0;

the identification system for identifying a regulation violation behavior of entering a dangerous area comprises: a regulation violation red line (803) and an entry sensor (811) for preventing regulation violation based on non-lock identification, wherein: the entry sensor (811) with a human body sensing probe is arranged on the regulation violation red line (803); and the entry sensor (811) continuously sends dangerous source information of the dangerous area, which is acting as regulation violation red line marker information, to the cloud computing center (7) for preventing regulation violation, to form a logical variable X6; in a case where the regulation violation red line marker information is received, X6=1, otherwise X6=0;

the identification system for identifying unmanned vehicle dangerous source comprises a dangerous source speed probe and a crash sensor (900) for preventing regulation violation based on non-lock identification, wherein: the dangerous source speed probe and the crash sensor (900) are arranged on an unmanned vehicle (905), and the crash sensor (900) transmits dangerous source information of the unmanned vehicle (905) to the microprocessor (15) through a port of a dangerous source information acquisition system of the intelligent sensor system for preventing regulation violation; the dangerous source information is processed by the microprocessor (15), and then sent from the 5G information converter (5) to the cloud computing center (7) for preventing regulation violation, to form a moving vehicle dangerous source logical variable X7; in a case where a speed reaches a danger value, the logical variable X7=1, otherwise, X7=0;

the identification system for identifying a regulation violation behavior of entering a rail transport lane where a pedestrian is not admitted comprises a vehicle power control switch (107) and a pedestrian sensor (105) for preventing regulation violation based on non-lock identification, wherein: the pedestrian sensor (105) is arranged and connected on the vehicle power control switch (107); the pedestrian sensor (105) is configured to transmit rail transport dangerous source information to the microprocessor (15) through a port of a dangerous source information acquisition system of the intelligent sensor system for preventing regulation violation; the rail transport dangerous source information is processed by the microprocessor (15) and sent from the 5G information converter (5) to the cloud computing center (7) for preventing regulation violation; and the cloud computing center (7) for preventing regulation violation takes an on/off state signal of the vehicle power control switch (107) as a dangerous source logical variable X8; in a case where the switch is on, it is indicated that the rail transport vehicle (103) is in danger of crash into a person during moving, X8=1, otherwise, X8=0.

21. The method of identifying a regulation violation behavior according to claim 19, wherein the regulation violation behavior identification system of device-based lock mechanism comprises a sensor circuit board (301), a sensor housing (303), a lock cylinder (305), a lossless connection sleeve (307), and a jackscrew (309), wherein: the lossless connection sleeve (307) is arranged on a bolt head of a fastening bolt (311) of a housing cover plate (313) of a device and then fixed onto a head of the fastening bolt (311) with the jackscrew (309); the sensor circuit board (301) is provided on one side inside the sensor housing (303); the sensor circuit board (301) is in signal connection with a microswitch node or a Hall element active switch; the other side inside the sensor housing (303) is arranged on the lossless connection sleeve (307); the lock cylinder (305) is arranged in a hole provided on one sidewall of the sensor housing (303), and fixes the sensor housing (303) onto the housing cover plate (313)of the device;

in a case where a full-time person removes the lock cylinder (305) by using a special key and then removes the sensor housing (303), the microswitch on the sensor circuit board (301) is triggered, action information of the switch is processed by the sensor circuit board (301), sent to a mining cloud computing center (201) for preventing regulation violation, processed and converted into an behavior logical variable B11, wherein: in a case where the sensor housing is removed, B11=1, otherwise B11=0; or

the regulation violation behavior identification system of device-based lock mechanism comprises a sensor circuit board (301), a sensor housing (303), a lossless connection lock cylinder (521), a lossless connection nut (512), a lossless connection bolt (513), a lossless connection bolt sleeve (515), a lossless connection cover (523), and a lock sleeve (527), wherein: the lossless connection cover (523) is arranged on a device cover plate (525); the lossless connection nut (512) is fixed onto the lossless connection cover (523); the lossless connection bolt (513) is sleeved with the lossless connection bolt sleeve (515) and screwed into the lossless connection nut (512) to fasten the lossless connection cover (523) onto the device cover plate (525); the lock sleeve (527) is arranged onto the lossless connection bolt sleeve (515); the lock sleeve (527) is fixed onto the lossless connection bolt sleeve (515) by screwing a key into the lossless connection lock cylinder (521); the sensor housing (303) is connected with the lock sleeve (527) to form an inner cavity of the sensor housing (303); the sensor circuit board (301) is arranged on the inner cavity of the sensor housing (303); an electric lock (517) of an electromagnet or a stepping motor is arranged on the lock sleeve (527); an acousto-optic identification probe (509) and a card reader (510) are arranged on one side surface of the sensor housing (303); and the sensor circuit board (301) is connected with the electric lock (517), the acousto-optic identification probe (509), and the card reader (510);

after the sensor circuit board (301) obtains voice or an image of an operator through the acousto-optic identification probe (509), or obtains personal code of a radio frequency card or a magnetic card through the card reader (510), the voice or image or personal code is transmitted to the microprocessor through a port of the regulation violation behavior identification system of device-based lock mechanism of the intelligent sensor system for preventing regulation violation, and sent from the 5G information converter to the mining cloud computing center (201)for preventing regulation violation; the mining cloud computing center (201) for preventing regulation violation enables the regulation violation behavior identification software for computation; and in a case where the operator is determined to be a full-time person, an instruction issued by the mining cloud computing center (201) is transmitted from the 5G information converter to the microprocessor (15) for processing, for commanding the electric lock (517) to act to open a passage, to enable the key (519) to operate the lock cylinder (521) for unlocking to remove the sensor housing (303); and in a case where the sensor housing (303) is removed, information is transmitted from the sensor circuit board (301) to the mining cloud computing center (201) for preventing regulation violation, and converted into an behavior logical variable B12; in a case where the sensor housing is removed, B12=1, otherwise B12=0.

22. The method of identifying a regulation violation behavior according to claim 19, wherein the regulation violation behavior identification system based on non-lock mechanism comprises an identification system for identifying a regulation violation behavior of drowsiness, an identification system for identifying a regulation violation behavior of not-watching-computer, an identification system for identifying a regulation violation behavior of hazardous gas leakage, an identification system for identifying a regulation violation behavior of entering a dangerous area, an identification system for identifying a regulation violation behavior of an unmanned vehicle (905), and an identification system for identifying a regulation violation behavior of entering a rail transport lane where a pedestrian is not admitted;

the identification system for identifying a regulation violation behavior of drowsiness comprises a drowsiness sensor (603) for preventing regulation violation based on non-lock identification and an preventing drowsiness means (607), wherein: the drowsiness sensor (603) is arranged on a conveyor magnetic starter (601) controlling a conveyor; the conveyor magnetic starter (601) is connected with the drowsiness sensor (603) through a connecting wire (602); the drowsiness sensor (603) is connected with the preventing drowsiness means (607) and wirelessly connected with the cloud computing center (7) for preventing regulation violation; and the cloud computing center (7) for preventing regulation violation is directly connected with a personnel positioning system (611) for data calling and sharing; in a case where the operator works normally, the preventing drowsiness means (607) is operated regularly; in a case where the operator is incapable of working normally due to regulation violation behavior of drowsiness, the operation of the preventing drowsiness means (607) is automatically stopped and drowsiness state information is sent to the drowsiness sensor (603), processed by the microprocessor (15) and sent from the 5G information converter (5) to the cloud computing center (7), to form a drowsiness logical variable B21; in a case where the operator is drowsy, B21=1, otherwise B21=0;

the identification system for identifying a regulation violation behavior of not-watching-computer comprises a not-watching-computer probe capable of regularly sending a sensing signal for preventing regulation violation, wherein: the not-watching-computer probe capable of regularly sending a sensing signal is arranged on a computer and connected with the intelligent sensor system for preventing regulation violation; in a case where an operator observes regulation to watch computer, the not-watching-computer probe regularly sends a signal to the intelligent sensor system for preventing regulation violation; in a case where the operator violates regulation not to watch computer, the not-watching-the-computer probe sends another signal, which is processed by the microprocessor (15) and transmitted from the 5G information converter (5) to the cloud computing center for preventing regulation violation, to form a logical variable B22 after comparison computation; in a case where the operator does not watch computer, B22=1, otherwise B22=0;

the identification system for identifying a regulation violation behavior of hazardous gas leakage comprises a manual valve leakage sensor (701) for preventing regulation violation based on non-lock identification and an electric valve leakage sensor (709) for preventing regulation violation based on non-lock identification, wherein: the manual valve leakage sensor (701) and the electric valve leakage sensor (709) are arranged at two positions and are both connected to the cloud computing center (7) for preventing regulation violation through their 5G information converters (5) for wireless communication; and the manual valve leakage sensor (701) is connected to one hazardous gas probe and one human body sensing probe; in a case where time of hazardous gas leakage caused by regulation violation is more than a certain time, the human body sensing probe of the manual valve leakage sensor (701) transmits information of whether there is an operator on site to the microprocessor (15); the information is processed and wirelessly reported to the cloud computing center (7) for preventing regulation violation for comparison computation, to form a logical variable B23; in a case where an operator is on site, B23=1, otherwise B23=0;

the identification system for identifying a regulation violation behavior of entering a dangerous area comprises a regulation violation red line (803) and an entry sensor (811) for preventing regulation violation based on non-lock identification, wherein: the entry sensor (811) is arranged on the regulation violation red line; in a case where the operator touches the regulation violation red line (803), the entry sensor (811) sends regulation violation behavior information of entering a dangerous area to the cloud computing center (7) for preventing regulation violation through a 5G information converter (5) corresponding to the sensor, to form a logical variable B24; in a case where the operator touches the regulation violation red line, B24=1, otherwise B24=0;

the identification system for identifying a regulation violation behavior of an unmanned vehicle comprises a human body sensing radar probe and a crash sensor (900) for preventing regulation violation based on non-lock identification, wherein: the human body sensing radar probe is arranged on the crash sensor (900); and the human body sensing radar probe and the crash sensor (900) are arranged on an unmanned vehicle (905); the crash sensor (900) transmits, to the microprocessor, behavior information of a pedestrian (903) who violates regulation to enter a road that is detected by the human body sensing radar probe; the behavior information is sent from the 5G information converter to the cloud computing center (7) for preventing regulation violation, to form a regulation violation logical variable B25; in a case where a distance between the pedestrian (903) who violates regulation to enter a road and the unmanned vehicle (905) approaches a specified value,B25=1, otherwise B25=0; and

the identification system for identifying a regulation violation behavior of entering a rail transport lane where a pedestrian is not admitted comprises a vehicle power control switch (107), a pedestrian sensor (105) for preventing regulation violation based on non-lock identification, and a laser (109), wherein: the pedestrian sensor (105) is arranged on the vehicle power control switch (107) and connected with the laser (109); laser emitted by the laser (109) is a regulation violation red line; in a case where a person (100) who violates regulation to enter a rail transport lane performs regulation violation behavior of shielding the laser, the pedestrian sensor (105) transmits regulation violation behavior information of the person to the microprocessor; the regulation violation behavior information is sent from the 5G information converter to the cloud computing center (7) for preventing regulation violation, to form a rail pedestrian regulation violation logical variable B26; in a case where the regulation violation red line is touched, B26=1, otherwise B26=0.

23. The method of identifying a regulation violation behavior according to claim 19, wherein the identity identification system (11) comprises an acoustic-optical identification probe (509), a card reader (510), and information of voice, an image, or a radio frequency card number profile identity of a full-time person that is pre-installed in the cloud computing center for preventing regulation violation, wherein: the acoustic-optical identification probe (509) and the card reader (510) are connected with the sensor circuit board; the acoustic-optical identification probe (509) and the card reader (510) transmit acquired actual identity information of the operator to the microprocessor (15); the acquired actual identity information being processed, and then sent from the 5G information converter (5) to the cloud computing center (7) for preventing regulation violation, and then compared with the profile information of the full-time person that is pre-installed in the cloud computing center (7) for preventing regulation violation to identify an operator identity, to form a logical variable C; in a case where the operator is full-time person, C=1, otherwise C=0; an identification result C=1 or C=0 is transmitted from the 5G information converter (5) to the microprocessor (15) to command the electric lock of the electric lock mechanism system (9) to act.

24. The method of identifying a regulation violation behavior according to claim 19, wherein the position and environment identification system comprises a database of the cloud computing center (7)for preventing regulation violation where information of device performance and sensor position profile information are stored, and an environmental temperature and photosensitive probe device arranged and connected on the intelligent sensor system for preventing regulation violation, wherein: the position and environment identification system regularly acquires related parameter information of a sensor position and a device surrounding temperature and transmits the information to the microprocessor (15); the information is sent from the 5G information converter to the cloud computing center (7) for preventing regulation violation, to form a position and environment identification logical variable D; and after the cloud computing center (7) for preventing regulation violation performs analysis and computation, a result of the analysis and computation is sent to a computer or a mobile phone terminal (6); in a case where the related parameter information is not zero: D=1, otherwise D=0; and

the auxiliary variable E identification system (13) comprises a switch comprehensive protection means and a radio frequency card reader, wherein: the auxiliary variable E identification system regularly acquires related parameter information of device voltage, device current, device short-circuit protection setting value, device leakage protection action, device power factor, and personnel patrol inspection and transmits the information to the microprocessor (15); the information is processed and then transmitted from the 5G information converter to the cloud computing center (7) for preventing regulation violation, to form an auxiliary variable logical variable E; and after analysis and computation by the cloud computing center (7) for preventing regulation violation, a result of the analysis and computation is transmitted to a computer or a mobile phone terminal (6); in a case where the related parameter information of the auxiliary variable logical variable E is not zero, E=I, otherwise E=0.

25. The method of identifying a regulation violation behavior according to claim 19, wherein the actuator system is any of the electric lock mechanism system (9), a superior power switch sensor (215) for preventing regulation violation, a pipeline electric valve (707), a possible rear-ending vehicle (907), a vehicle power control switch (107), or a mobile phone of an emergency rescuer (807);

the electric lock mechanism system (9) comprise an electro-magnet electric lock or stepping motor electric lock, wherein the electro-magnet electric lock or stepping motor electric lock (517) is arranged on a lock sleeve (527); an electric lock action state signal is sent to the cloud computing center (7) for preventing regulation violation through the microprocessor (15) and the 5G information converter (5) in succession, to form a logical variable H; in a case where the electric lock is opened, H=1; and in a case where the electric lock is closed, H=0; the cloud computing center (7) for preventing regulation violation performs identification computation after receiving a signal sent from the identity identification system (11); in a case where the person is considered a full-time person, H=1 is set and the electric lock is commanded to be opened, otherwise, H=0 is set and the electric lock is commanded to be closed;

the superior power switch sensor (215) for preventing regulation violation is arranged on a load switch (209), wherein a load switch sensor (205) for preventing regulation violation issues a power cut or locking request to an superior power switch (213) controlling a dangerous source facility; the request is computed and processed by a mining cloud computing center (201) for preventing regulation violation, for commanding the superior power switch sensor (215) to issue a power cut instruction, to command the superior power switch (213) to be powered off or locked; while the superior power switch (213) transmits power to the load switch (209), live information is transmitted from the superior power switch sensor (215) to the mining cloud computing center (201) for preventing regulation violation and converted into a voltage dangerous source logical variable X1; in a case where there is live, X1=1, otherwise X1=0; in a case where X1=1, the mining cloud computing center (201) for preventing regulation violation synchronously commands an electric lock of the load switch sensor (205) for preventing regulation violation to be locked, to disable the operator to perform a regulation violation behavior of uncapping operation, for identifying and locking a live dangerous source; in a case where X1=0, the mining cloud computing center (201) for preventing regulation violation synchronously commands the electric lock of the load switch sensor (205) to be unlocked, to enable the operator to perform an uncapping operation; and

the pipeline electric valve (707), the possible rear-ending vehicle (907), the vehicle power control switch (107), or the mobile phone of the emergency rescuer (807) is a facility for controlling the dangerous source; after the cloud computing center (7) for preventing regulation violation determines a regulation violation and sends an instruction to the facility for controlling the dangerous source, the facility for controlling the dangerous source is commanded to act, to cut off and lock the dangerous source, and meanwhile, give an alarm.

26. The method of identifying a regulation violation behavior according to claim 19, wherein the regulation violation identification algorithm comprises at least one of :

$$W11=X1*(B1+B2)*C=1;$$

$$W12=X2*(B1+B2)*C=1;$$

$$W1n=Xn*(B1+B2)*C=1;$$

$$W21=X1*(B1+B2)=1;$$

$$W22=X2*(B1+B2)=1;$$

$$W2n=Xn*(B1+B2)=1;$$

$$W3=f(X,B1,B2,C,D,E,H)=1;$$

or

$$FW=(B1+B2)*FX*FC=1;$$

where X1, X2 and Xn represent first, second and nth dangerous source logical variables, W with a serial number represents various regulation violation behavior logical variables, FW represents a non-regulation violation behavior logical variable, FX represents a non-dangerous source logical variable, and FC represents a non-full-time person logical variable.

27. The method of identifying a regulation violation behavior according to claim 19, wherein the cloud computing center for preventing regulation violation is further capable of being networked with BeiDou Navigation System, GPS Navigation System, an underground personnel positioning system, or a methane monitoring system, for data calling and sharing.

28. An intelligent sensor system of preventing regulation violation for performing the method according to claim 19, comprising a regulation violation behavior identification system (1) based on lock mechanism, a regulation violation behavior identification system (3) based on non-lock mechanism, a 5G information converter (5), a computer or a mobile phone terminal (6) installed with regulation violation behavior identification and processing software, a cloud computing center (7) for preventing regulation violation installed with the regulation violation behavior identification and processing software, an electric lock mechanism system (9), an identity identification system (11), an auxiliary variable E identification system (13), a microprocessor (15), a position and environment identification system (17), and a dangerous source information acquisition system (19), wherein: an information input end of the microprocessor (15) is connected with the regulation violation behavior identification system (1)based on lock mechanism, the regulation violation behavior identification system (3) based on non-lock mechanism, the dangerous source information acquisition system (19), the identity identification system (11), the position and environment identification system (17), and the auxiliary variable E identification system (13); a control signal output end of the microprocessor (15) is connected with signal input ends of the electric lock mechanism system (9) and the 5G information converter (5); a signal output end of the 5G information converter (5) is wirelessly connected with the cloud computing center (7) for preventing regulation violation; and the cloud computing center (7) for preventing regulation violation is connected with the computer or the mobile phone terminal (6); and

a basic workflow of the intelligent sensor system for preventing regulation violation comprises: starting; initializing; reading EEPROM configuration information; configuring the 5G information converter (5); connecting a 5G network; receiving a command from the cloud computing center (7)for preventing regulation violation and parsing the command; regularly reporting information of the dangerous source information acquisition system (19), information of the position and environment identification system (17), and information of the auxiliary variable E identification system (13); and circularly detecting information of the regulation violation behavior identification system (1) based on lock mechanism, information of the regulation violation behavior identification system (3) based on non-lock mechanism, information of the identity identification system (11), and electric lock action information of the electric lock mechanism system (9).

Fig.1

Fig.2

Fig.3

Fig.4

Fig.5

Fig.6

Fig.7

Fig.8

Fig.9

Fig.10

starting

↓

initializing

↓

reading EEPROM configuration information

↓

reading successfully

— not → using default configuration

↓ yes

configuring 5G module to be connected to cloud computing center

↓

connecting successfully

— not →

↓ yes  ①

receiving cloud computing center command

— not → regularly reporting dangerous source, position and environment, voltage and current and other information

↓ yes

parsing control command, executing control command, reporting information of facility, and waiting for cloud computing center to read

↓ yes

if lock mechanism regulation violation identification information is detected

— yes → reporting to cloud computing center to wait for reading

↓ not

②

Fig.11a

Fig.11b

obtain dangerous source state information and behavior information of an operator — S1210

determine whether the operator has a regulation violation behavior according to the dangerous source state information and the behavior information of the operator — S1220

in a case where the operator has the regulation violation behavior, command an actuator system to execute a first action — S1230

Fig.12

— 1300

obtaining module — 1310

determining module — 1320

control module — 1330

Fig.13

1400

acquisition
device — 1410

cloud
computing
center — 1420

Fig.14

| | | |
|---|---|---|
| **INTERNATIONAL SEARCH REPORT** | International application No. | |
| | **PCT/CN2022/109735** | |

**A. CLASSIFICATION OF SUBJECT MATTER**

G01D 21/02(2006.01)i; G01N 33/00(2006.01)i; G01S 17/931(2020.01)i; G01S 19/42(2010.01)i; G06K 17/00(2006.01)i; H04W 4/38(2018.01)i

According to International Patent Classification (IPC) or to both national classification and IPC

**B. FIELDS SEARCHED**

Minimum documentation searched (classification system followed by classification symbols)

G01D G01N G01S G06K H04W

Documentation searched other than minimum documentation to the extent that such documents are included in the fields searched

Electronic data base consulted during the international search (name of data base and, where practicable, search terms used)

CNABS; CNTXT; CNKI; CJFD; 万方, WANFANG; 超星, CHAOXING; VEN; USTXT; EPTXT; WOTXT; IEEE: 山西全安新技术开发有限公司, 郭春平, 郭晓鹏, 违章, 违规, 不安全, 危险, 动作, 行为, 源, 状态, 作业, 人, 执行, 有害, 气, 物, 污染, 开盖, 阈值, 设定, 预设, 闭锁, 开机, 停机, 离岗, 脱岗, violation, person, people, behavior, danger+ , gas, leakage

**C. DOCUMENTS CONSIDERED TO BE RELEVANT**

| Category* | Citation of document, with indication, where appropriate, of the relevant passages | Relevant to claim No. |
|---|---|---|
| PX | CN 113819953 A (SHANXI QUAN'AN HIGH TECHNOLOGY DEVELOPMENT CO., LTD.) 21 December 2021 (2021-12-21)<br>description, paragraphs [0001]-[0126], and figures 1-12 | 1-28 |
| X | CN 112837010 A (WUHAN MARINE MACHINERY PLANT CO., LTD.) 25 May 2021 (2021-05-25)<br>description, paragraphs [0004]-[0089], and figures 1-2 | 1-18 |
| X | CN 112153345 A (INNER MONGOLIA MENGDA POWER GENERATING CO., LTD. et al.) 29 December 2020 (2020-12-29)<br>description, paragraphs [0002]-[0067], and figures 1-4 | 1-18 |
| A | CN 112261384 A (HUANENG (FUJIAN, ZHANGZHOU) ENERGY CO., LTD.) 22 January 2021 (2021-01-22)<br>entire document | 1-28 |
| A | CN 111813841 A (HUADIAN XINZHOU GUANGYU COAL POWER CO., LTD. et al.) 23 October 2020 (2020-10-23)<br>entire document | 1-28 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **29 September 2022** | **24 October 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/109735** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 204082213 U (SHANXI QUAN'AN HIGH TECHNOLOGY DEVELOPMENT CO., LTD.) 07 January 2015 (2015-01-07)<br>entire document | 1-28 |
| A | WO 2012150591 A2 (ATSMON, A. et al.) 08 November 2012 (2012-11-08)<br>entire document | 1-28 |

Form PCT/ISA/210 (second sheet) (January 2015)

INTERNATIONAL SEARCH REPORT
Information on patent family members

International application No.

**PCT/CN2022/109735**

| Patent document cited in search report | | | Publication date (day/month/year) | Patent family member(s) | | | Publication date (day/month/year) |
|---|---|---|---|---|---|---|---|
| CN | 113819953 | A | 21 December 2021 | None | | | |
| CN | 112837010 | A | 25 May 2021 | None | | | |
| CN | 112153345 | A | 29 December 2020 | None | | | |
| CN | 112261384 | A | 22 January 2021 | None | | | |
| CN | 111813841 | A | 23 October 2020 | None | | | |
| CN | 204082213 | U | 07 January 2015 | None | | | |
| WO | 2012150591 | A2 | 08 November 2012 | US | 2014111647 | A1 | 24 April 2014 |
| | | | | EP | 3416153 | A1 | 19 December 2018 |
| | | | | EP | 2705664 | A2 | 12 March 2014 |
| | | | | US | 2016371551 | A1 | 22 December 2016 |
| | | | | WO | 2012150591 | A3 | 02 May 2013 |
| | | | | US | 9443152 | B2 | 13 September 2016 |
| | | | | US | 10147004 | B2 | 04 December 2018 |
| | | | | IL | 229214 | A | 28 February 2018 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111213708

- CN 200810055240

**Non-patent literature cited in the description**

- REGULATION VIOLATION BEHAVIOR ANALYSIS AND PREVENTING REGULATION VIOLATION COUNTERMEASURES. Peking University, 2017